# EUROPEAN PATENT SPECIFICATION

(11) **EP 2 099 289 B1**
(45) Date of publication and mention of the grant of the patent: **18.03.2015**
(21) Application number: 08701746.3
(22) Date of filing: 07.01.2008
(51) Int. Cl.: A01K 67/00, C12N 15/00

(54) **HUMANISATION OF ANIMALS**
HUMANISIERUNG VON TIEREN
HUMANISATION D'ANIMAUX

(30) Priority: 05.01.2007 GB 0700194
(43) Date of publication of application: 16.09.2009
(73) Proprietor: The University Court of the University of Edinburgh, Edinburgh EH8 9YL (GB)
(72) Inventor: SMITH, Andrew, John, Hammond, Edinburgh EH3 6NT (GB); WALLACE, Helen, Anne, Chapman, Edinburgh EH10 6SH (GB); HIGGS, Douglas, Roland, Oxford OX3 9DS (GB)
(74) Representative: Goodfellow, Hugh Robin
(86) International application number: PCT/GB2008/000028
(87) International publication number: WO 2008/081197

(56) References cited:
- WO-A-2004/076618
- ANDRÉ OUMARD ET AL: "Recommended Method for Chromosome Exploitation: RMCE-based Cassette-exchange Systems in Animal Cell Biotechnology" CYTOTECHNOLOGY, KLUWER ACADEMIC PUBLISHERS, DO, vol. 50, no. 1-3, 14 June 2006 (2006-06-14), pages 93-108, XP019393904 ISSN: 1573-0778
- LIU KAI ET AL: "Recombinase-mediated cassette exchange to rapidly and efficiently generate mice with human cardiac sodium channels" GENESIS THE JOURNAL OF GENETICS AND DEVELOPMENT, vol. 44, no. 11, November 2006 (2006-11), pages 556-564, XP002481711 ISSN: 1526-954X
- AL-HASANI KEITH ET AL: "Complementation of alpha-thalassaemia in alpha-globin knockout mice with a 191 kb transgene containing the human alpha-globin locus" TRANSGENIC RESEARCH, vol. 13, no. 3, June 2004 (2004-06), pages 235-243, XP002481712 ISSN: 0962-8819
- WALLACE HELEN A C ET AL: "Manipulating the mouse genome to engineer precise functional syntenic replacements with human sequence" CELL, vol. 128, no. 1, January 2007 (2007-01), pages 197-209, XP002481713 ISSN: 0092-8674
- BRANDT WILLIAM ET AL: "Defining the functional boundaries of the Gata2 locus by rescue with a linked bacterial artificial chromosome transgene", JOURNAL OF BIOLOGICAL CHEMISTRY, vol. 283, no. 14, April 2008 (2008-04), pages 8976-8983, ISSN: 0021-9258
- KUROIWA Y ET AL: "MANIPULATION OF HUMAN MINICHROMOSOMES TO CARRY GREATER THAN MEGABASE-SIZED CHROMOSOME INSERTS", NATURE BIOTECHNOLOGY, NATURE PUBLISHING GROUP, NEW YORK, NY, US, vol. 18, 1 October 2000 (2000-10-01), pages 1086-1090, XP002949330, ISSN: 1087-0156, DOI: 10.1038/80287
- ZHANG XIN-MEI ET AL: "Combination of overlapping bacterial artificial chromosomes by a two-step recombinogenic engineering method.", NUCLEIC ACIDS RESEARCH 1 AUG 2003 LNKD- PUBMED:12888533, vol. 31, no. 15, 1 August 2003 (2003-08-01), page e81, ISSN: 1362-4962
- Z. Larin ET AL: "Yeast artificial chromosome libraries containing large inserts from mouse and human DNA.", Proceedings of the National Academy of Sciences, vol. 88, no. 10, 15 May 1991 (1991-05-15), pages 4123-4127, XP055111458, ISSN: 0027-8424, DOI: 10.1073/pnas.88.10.4123

## Description

### Field of the Invention

The present invention relates to the generation of transgenic animal cells and/or animals in which a large portion of a host animal's genome has been replaced with an equivalent syntenic portion of nucleic acid from the genome of a different organism.

### Background to the Invention

Studies of how human genes are normally regulated and the effects of mutations that cause human genetic disease are limited by the availability of appropriate primary cells and tissues and the necessary constraints on experimental interventions. Consequently, over the past 20 years, the mouse has become a major experimental model to understand how human genes are regulated during differentiation and development and to discover the mechanisms by which mutations in critical genes give rise to specific disease phenotypes.

To date, the most thorough way to address how human genes are regulated *in vivo* has been to make transgenic mouse models. However, small transgenes often do not contain all of the *cis*-acting elements required for fully regulated expression since such sequences may be found tens or even hundreds of kilobases from the gene in question (Kleinjan and van Heyningen, 2005). Even when large transgenes derived from PACs, BACs or YACs are used, they are frequently rearranged; it is often very difficult to fully analyse their structural integrity and copy number (Peterson et al., 1998). Furthermore, their expression is often influenced by their position of integration in the genome (Alami et al., 2000; Kaufman et al., 1999). Making and characterising directed mutations for structure/function studies is also difficult when using such large molecules. Even when successful, the interpretation of these transgenic experiments is complicated by the fact that the endogenous mouse genes are still present. To circumvent this problem the transgenic mice have to be bred against mice in which the endogenous gene has been deleted or inactivated by homologous recombination. A complementary approach is to identify the mouse orthologue and alter this gene or its regulatory elements by homologous recombination using conventional gene targeting methods (Nagy, 2003). Clearly, mutating a single gene at its normal chromosomal locus avoids many of the problems associated with transgenes (copy number differences, complex mapping problems, position effects) and, in this process, the normal endogenous gene does not remain intact to complicate the analysis. However, this approach is not without its own problems since there is increasing evidence demonstrating considerable differences in the ways in which orthologous human and mouse genes are normally regulated, with altered phenotypes between the species when mutated (Anguita et al., 2002). In many instances mutations known to cause disease in human do not mimic the phenotype when introduced into the mouse orthologue (Colledge et al., 1995; Engle et al., 1996; Garrick et al., 2006). Therefore, although mouse models have provided many insights into human genetic disease, it is becoming increasingly clear that they are also beset by inherent problems and difficulties in interpretation resulting from differences in basic biological processes that have evolved over the 70 million years of evolution that separate humans and mice.

Clearly, the next step in developing mouse models of human disease is to replace large segments (100-1000s kb) of the mouse genome with the wild type or mutated syntenic region of the human sequence thereby including all remote regulatory elements and inserting the human segment as a single copy at a natural chromosomal position for the genes contained within it. A range of chromosome engineering technologies are now available that, when carefully selected and combined in a novel way, could make this feasible (Yu and Bradley, 2001) (Copeland et al., 2001; Muyrers et al., 2001) (Testa et al., 2003; Valenzuela et al., 2003; Yang and Seed, 2003), including application of various heterotypic site-specific recombination systems (Cre, Flp, PhiC31) to achieve efficient exchange of sequence cassettes at targeted chromosomal loci, a process known as recombinase-mediated cassette exchange (RMCE) (Baer and Bode, 2001).

The present invention, which is based on work by the present inventors, enables the replacement of large segments of a host's genome with a syntenic region from a different organism, using site-specific recombination, while ensuring very little extraneous nucleic acid is left at the sites of recombination in the host genome. The invention, which incorporates some aspects of the above methods, provides a universally applicable means for replacement of a host's genome with any desired sequence, in principle unrestricted by the size of sequence, by site-specific recombination.

The present inventors have demonstrated this new, general strategy by replacing large segments of the mouse genome with wild type or mutated syntenic regions of the human genome. The inventors refer to this process as recombinase-mediated genomic replacement (RMGR). This allows precise, reproducible, genetically selectable genomic replacement on a scale not previously attained and should be generally applicable to large scale modification of, for example, the mouse genome. The inventors have 'humanised' mice by replacing the syntenic region encompassing the mouse α globin regulatory domain (the region containing all *cis*-acting sequences required for fully regulated expression of the α globin genes) with human BAC-derived sequence in ES cells and subsequently deriving viable mice homozygous for this syntenic substitution producing only human α-globin chains. In addition, the inventors have demonstrated the potential of this approach for making authentic mouse models of human disease by replacing the mouse region with a recombineered human BAC in which the α globin major regulatory element has been deleted from the α globin regulatory domain. This created a model of α thalassemia that faithfully mimics the phenotype of patients with this common human genetic disease.

WO04076618 describes a strategy for 'humanisation' involving creation of a deletion of the desired genomic region and substitution with human sequence either at the site of deletion or elsewhere in the genome. This is described in the context of the 'humanisation' in ES cells and mice of the entire immunoglobulin heavy chain constant region. WO04076618 describes how by successive rounds of gene targeting *lox* sites can be inserted into the positions of the most terminal 5' and 3' constant region genes and consequently on expression of Cre recombinase a deletion of all the constant region genes can be achieved. Data is presented demonstrating proof of the deletion strategy. It should be noted that at the time of filing in 2003 there was nothing novel about Cre-mediated long range deletions (several examples, including Smith A.J.H., et al. 2002 Cre-loxP chromosome engineering of a targeted deletion in the mouse corresponding to the 3p21.3 region of homozygous loss in human tumours. Oncogene 21: 4521-45299 had been described. The application then goes onto state how such cells can be a route to creating humanised mice by one of several possible methods. In the most conceptually simple way this is by crossing with mice carrying a YAC or BAC chromosomal integration with the relevant human sequence. The second method is to include human sequences in the initial targeting constructs in such a position relative to the *lox* sites that after Cre-mediated deletion these are left integrated and their expression activated. The third way (not explained in any detail) is by insertion of human sequences via the *lox* site remaining at the deletion point in a subsequent step. It should be noted that in WO04076618 no data is given to demonstrate the practicality of the last two methods in the context of the deletion they have created (these are only mentioned as being possibly based on other published work or as being 'in principle' possible).

As will be understood from a full understanding of the present invention, it distinct from and clearly superior to that disclosed in WO04076618.

### Method 1. Inter-breeding the deletion strain with a strain carrying a human BAC (or YAC) integration made by conventional pronuclear injection.

There are several disadvantages: (i) creating a large multi-gene deletion may result in a haplo-insufficiency phenotype (a consequence of reduced gene dosage) in either the ES cells (not very likely) or in mice (more likely); (ii) inter-breeding involves additional time - first you need to create the compound heterozygous strain [+/deletion; +BAC] and then intercross to create the compound homozygous strain [deletion/deletion; +BAC or BACBAC]; (iii) most importantly the region contained within the BAC (or YAC) integration is very unlikely to match precisely the region deleted - a consequence of terminal end DNA deletion/rearrangement during random chromosomal integration in conventional BAC/YAC transgenesis.

The present invention seeks to obviate and/or mitigate at least one of the aforementioned disadvantages.

### Summary of the Invention

There is described herein a transgenic animal cell in which a portion of the genome of the cell has been replaced by an equivalent portion of nucleic acid sequence from a region of synteny of a different organism, wherein the nucleic acid which has been replaced using site-specific recombination is greater than 50 kb in size and the resulting genome following replacement of the nucleic acid, is substantially free of extraneous nucleic acid at or adjacent to the two end points of the replacement interval.

Typically the animal cell is an embryonic stem cell which is capable of, through microinjection of a blastocyst, being used to generate a transgenic animal having cells comprising the replaced nucleic acid. Initial animals generated may be heterozygous for the replaced nucleic acid, but after appropriate cross-breading of such heterozygous animals, animals which are homozygous for the replacement can be obtained.

A homozygous transgenic animal is described herein, which comprises cells in which a portion of the genome has been replaced by an equivalent portion of nucleic acid from a region of synteny of a different organism, wherein the nucleic acid which has been replaced is greater than 50 kb in size and the resulting genome following replacement, is substantially free of extraneous nucleic acid at or adjacent to the two end points of the replacement interval.

Although the invention is generally applicable to replacement of nucleic acid from the genome of any animal, with nucleic acid from any other animal, it is most desirable that the animal or animal cell is a mouse, rat, guinea pig, dog, cat or primate, wherein the portion of replaced genome is from a human. In this manner, the animal/animal cell can be thought of as being "humanised".

The present invention is applicable to the replacement of large syntenic portions of a genome with nucleic acid from a different organism. It is understood that the term "syntenic" refers to a region of chromosome from a first organism being relayed by a corresponding region from any other chromosome. For example, a region of chromosome encoding an immunoglobin gene cluster from one organism may be replaced with what is understood to be a corresponding immunoglobin gene cluster from another region. It is to be appreciated that the syntenic region to be replaced may comprise one or more deletions or mutations, SNPs etc., so their function can be studied it is also to be appreciated that the sizes of the two syntenic regions may not be similar in size and can vary considerably. Although mention is made to the replacement of at least 50 kb, this should not be construed as limiting and portions which may be replaced can be at least 100 kb, 200 kb, 500 kb, 1Mb, or even multi-Mb in size. This is achievable by use of appropriate site-specific recombination sites and not through general homologous recombination.

Site-specific recombination is not to be confused with general homologous recombination, which can occur between two DNA sequences that display regions of similarity or identity. Site-specific recombination differs from general homologous recombination in that short specific DNA sequences, which are required for the recombination, are the only sites at which recombination occurs. Moreover, site-specific recombination invariably requires specialised recombinases to recognise the sites and catalyse the recombination at these sites. A number of bacteriophage and yeast derived site-specific recombination systems, each comprising a recombinase and specific cognate sites, have been shown to work in eukaryotic cells for the purpose of DNA integration and are therefore applicable for use in the present invention, and these include the bacteriophage P1 Cre/lox and yeast FLP-FRT systems of the tyrosine family of site-specific recombinases. Other systems, of the tyrosine family such as bacteriophage lambda Int integrase, HK2022 integrase, and in addition systems belonging to the separate serine family of recombinases such as bacteriophage phiC31, R4 Tp901 integrases are known to work in mammalian cells using their respective recombination sites (Tronche, F. *et al.* 2002), and are also applicable for use in the present invention.

It is to be appreciated that the present invention is not to be construed as limited to the aforementioned systems, as other site-specific recombination systems may be used, even hitherto not identified systems, providing they can effectively recombine such large fragments of nucleic acid.

Advantageously, the present invention allows for the replacement of large portions of nucleic acid, whilst ensuring very little extraneous/non-essential nucleic acid is cloned into the host's genome, at or adjacent to the two end points of the replacement interval. Such extraneous/non-essential nucleic acid may have an effect on the functional expression of the replaced nucleic acid and its inclusion should desirably be avoided. Through the use of appropriate marker systems and site-recombination systems, the present inventors have been able to replace large portions of a genome with a syntenic region from a different organism without concomitant inclusion of substantial extraneous/non-essential nucleic acid at the sites of recombination. Typically any extraneous/non-essential nucleic acid which remains after nucleic acid replacement may be less than 100 bp or 50 bp and may comprise, for example, one or more restriction sites and/or site-specific recombination sites which are not removed following the replacement reaction. Such sequences can easily be discerned and identified by the skilled addressee and can be understood to not be part of the replaced region of synteny. It is to be appreciated that when replacing large portions of genome, inclusion of such small amounts of extraneous/non-essential nucleic acid is proportionately extremely low. In this regard, any extraneous/non-essential nucleic acid (i.e. not forming part of the region of synteny) included in the final genome may be typically less than 0.001% or 0.0001 % of the portion of nucleic acid genome replaced.

As described hereinafter in greater detail, a "humanised" mouse is provided, in which approximately 87 kb of mouse genome has been replaced with 117 kb of a syntenic portion of the human genome encoding the human a globin gene cluster. The mouse comprises very little extraneous nucleic acid at or adjacent to the recombination sites that is nucleic acid not already present in the mouse's genome prior to introduction of the site-specific recombination sites and/or replacement of the region of synteny, or indeed understood to be part of the region of synteny.

It is to be appreciated that creating transgenic animals, such as mice or rats, which comprise integrated copies of human genes to replace the equivalent mouse or rat genes can facilitate functional analyses of the genes in an animal model setting. Furthermore, when an introduced human gene(s) contains a known disease causing mutation, the associated pathology may be recapitulated.

The present invention allows for the study of mutational analysis of complex global regulatory regions by *in-vitro* differentiation of ES cells and/or in a model animal such as a mouse. Moreover, such a system offers the possibility for *in-vivo* screening of phenotypes associated with single nucleotide polymorphisms (SNPs) identified in human sequence to define for example potential quantitative trait loci, an application that could be relevant in pharmacogenomic studies. Also, "humanisation" of large gene clusters, such as immunoglobulin genes or major histocompatibility complex genes can allow the production of more accurate animal models of human immune disorders. It some embodiments, it may be desirable to retain some of the host orginisms genes when replacing a large region of nucleic acid from a different organism, as this may be required to, for example, enable correct expression of the region. The host's nucleic acid which is retained may be located internally of the nucleic acid replaced or at one or both ends thereof.

In accordance with the invention, there is provided methods for use in generating the animal cell or animal described herein.

A method is provided according to claim 1. In such a method, a non-human animal cell may be generated in which a single large fragment of at least 50 kb of the cell's genome has been replaced by an equivalent portion of nucleic acid sequence from a corresponding region from a chromosome of a different organism. The method may comprise the steps of:
a) providing an isolated non-human animal cell in the genome of which two site-specific recombination sites have been introduced that are not capable of recombining with each other, and which flank a portion of genome to be replaced with a corresponding region from a chromosome of a different organism,
b) providing a vector comprising said equivalent portion of genomic nucleic acid from the different organism flanked by the same two site-specific recombination sites that flank the portion of genome to be replaced in the cell, wherein the vector is a bacterial artificial chromosome (BAC), a PI artificial chromosome (PAC), a yeast artificial chromosome (YAC) or a mammalian artificial chromosome (MAC);
c) bringing said genome and said vector into contact *in vitro,* in combination with a site specific recombinase which is capable of recognising said two recombinase sites, such that under appropriate conditions a recombination event can occur which results in the equivalent portion of nucleic acid in the vector replacing the corresponding portion of genome from the cell; and
d) isolating a cell which comprises the replaced portion of nucleic acid.

Moreover, there is also described a method of generating a transgenic animal comprising cells in which at least 50 kb of the animal's genome has been replaced by an equivalent portion of nucleic acid sequence from a region of synteny of a different organism, comprising the steps of:
a) providing an animal embryonic stem cell having a genome that contains two site-specific recombination sites that are not capable of recombining with each other, and which flank a portion of genome to be replaced with an equivalent portion of nucleic acid sequence from a region of synteny of a different organism,
b) providing a vector comprising said equivalent portion of nucleic acid from the different organism flanked by the same two site-specific recombination sites that flank the portion of genome to be replaced in the embryonic stem cell;
c) bringing said animal embryonic stem cell and said vector into contact, in combination with a site specific recombinase, which is capable of recognising said two recombinase sites such that under appropriate conditions a recombination event can occur which results in the equivalent portion of nucleic acid in the vector, replacing the corresponding portion of genome in the embryonic stem cell;
d) isolating from the selection medium an embryonic stem cell which comprises the replaced portion of nucleic acid and using said embryonic stem cell to generate a chimaeric animal having cells which comprise said replaced nucleic acid; and
e) test-crossing said chimaeric animal with wild type mice so as to obtain germ line transmission of the genomic replacement and generate heterozygote progeny for said replaced nucleic acid, and
f) inter-crossing heterozygote progeny so as to generate homozygote progeny in which both chromosomal copies of the genome comprise the replaced nucleic acid.

In the above described methods, said two site-specific recombination sites are non-interacting (heterospecific) recombination sites such as *loxP* and a *low511* sites [or others such as *lox2272, low5171* (Lee, G. & Saito, I. 1998)]. It may also be desirable to introduce such site-specific recombination sites into introns, as opposed to coding or non-coding nucleic acid. This may avoid inadvertently disrupting any regulatory sequences on insertion of site-specific recombination sites into the genome of the animal cell. Conveniently introduction of the site-specific recombination sites may be achieved by conventional homologous recombination techniques. The skilled addressee is well aware of suitable conventional molecular biology techniques which may be employed to facilitate cloning of nucleic acid, introduction of necessary restriction or recombination sites, generation of transformed cells and/or transgenic animal production, but for the sake of completeness, the skilled reader is directed to the references mentioned hereinafter and to Sambrook and Russell (2001) (Molecular cloning: a laboratory manual 3rd edn (Cold Spring Harbor, N.Y.: Cold Spring Harbor Laboratory Press) and Nagy, A. (2003). (Manipulating the mouse embryo: a laboratory manual, 3rd edn (Cold Spring Harbor, N.Y.: Cold Spring Harbor Laboratory Press).

Introduction of the site-specific recombination sites into the genome can be achieved using vectors designed for positive-negative selection as known in the art and integration effected using homologous recombination.

The vector comprising said equivalent portion of nucleic acid must be capable of containing such large replacement fragments. Thus, the vector is typically a BAC, PAC, YAC or MAC vector known in the art respectively as a Bacterial, P1, yeast or mammalin artificial chromosome (Giraldo P. and Montoliu L. 2000; Sgaramella, V. and Eridani, S. 2004). In addition to the nucleic acid to be used to replace the equivalent nucleic acid in the animal cell, the vector also comprises the same two first site-specific recombination sites in equivalent positions and in the same orientation as in the animal cell.

In order to facilitate identification of cells which have undergone the replacement reaction, an appropriate genetic marker system may be employed and cells selected by, for example use of a selection medium. However, in order to ensure that the genome sequence is substantially free of extraneous nucleic at or adjacent to the two end points of the replacement interval, desirably the marker system/gene can be removed following selection of the cells containing the replaced nucleic acid.

Thus, in a preferred embodiment of the method of the present invention, cells in which the replacement of the portion of genome has taken place, may be positively selected by use of a marker gene, which can subsequently be removed from the cells. A system that may be used is based on the use of two non-functional portions of a marker gene, which are brought into functional association upon a successful replacement reaction being carried out and wherein the functionally reconstituted marker gene is flanked on either side by further site specific recombination sites (which are different to the site-specific recombination sites used for the replacement reaction), such that the marker gene can be excised from the genome, using an appropriate site-specific recombinase.

Thus, in a further aspect there is provided an *in vitro* method of generating a non-human animal cell in which a single large fragment of at least 50 kb of a region of a chromosome from the cell's genome has been replaced by a corresponding region from a chromosome of a different organism, comprising the steps of:
a) providing an isolated non-human animal cell in the genome of which two first site-specific recombination sites have been introduced that are not capable of recombining with each other, and which sites flank a portion of genome to be replaced with a corresponding region from a chromosome of a different organism, and further comprising a first inactive portion of a marker gene flanked 3' or 5' by a further second site-specific recombinase site;
b) providing a vector comprising said equivalent portion of nucleic acid from the different organism flanked by the same two first site-specific recombination sites that flank the portion of genome to be replaced in the cell and further comprising a second inactive portion of the marker gene flanked 5' or 3' respectively by said further different recombinase site;
c) bringing said genome and said vector into contact *in vitro,* in combination with a site specific recombinase which is capable of recognising said two first recombinase sites, such that under appropriate conditions a recombination event can occur which results in the equivalent portion of nucleic acid in the vector, replacing the corresponding portion of genome from the cell and whereby upon replacement of the portion of genome, said first and second inactive portions of the marker gene are brought into functional association, such that the replacement reaction can be selected for by use of a selection medium, whereby only cells which possess a functionally active marker gene and have been subject to the replacement reaction are able to survive in said selection medium;
d) isolating from the selection medium a cell which comprises the replaced portion of nucleic acid; and
e) contacting said cell with a recombinase that is capable of recognising said further second site-specific recombinase sites flanking said functional marker gene, such that site-specific recombination can occur, so as to delete the marker gene from the genome.

The recombinase may be provided as a purified protein, or a construct transiently expressed within the cell in order to provide the recombinase activity. Alternatively, the cell may be used to generate a transgenic animal, which may be crossed with an animal which expresses said recombinase, in order to produce progeny which lack the marker gene and associated recombination sites.

The method may comprise the
following additional steps:
f) isolating from the selection medium an embryonic stem cell which comprises the replaced portion of nucleic acid and using said cell to generate a chimaeric animal comprising cells which include the replaced nucleic acid and marker gene; and
g) crossing said chimaeric animal with an animal which expresses a recombinase that is capable of recognising said further second site specific recombinase sites flanking said functional marker gene, such that site specific recombination can occur, so as to delete the marker gene and site specific recombination sites from the genome.

Animals produced in accordance with the above described methods will be heterozygous for the replaced nucleic acid and as such, the methods may preferably further comprise:
f) crossing two heterozygote animals, so as to generate homozygote progeny in which both chromosomal copies of the genome comprise the replacement.

Preferably the first inactive portion of the maker gene is a 5' or 3' defective portion of a functional hypoxanthinephosphoribosyltransferase (HPRT) mini-gene, and the second inactive portion is a respective 3' or 5' defective further portion of said HPRT mini-gene. Depending on which of the two defective portions are to be introduced into the genome, the skilled reader will appreciate where the further second site-specific recombinase sites, are to be located 5' or 3' of its position. Conveniently the second site-specific recombinase sites are Frt sites, which are recognised by Flp recombinase, although other site-specific recombinase/site systems may also be used, providing they are different to those used in the replacement reaction. By removing the marker gene and recombination sites, very little extraneous nucleic acid may be left at or adjacent the original sites of recombination.

It should be further noted that ES cells could be generated so as to be homozygous for such a replacement following removal of the reconstructed HPRT gene by Flp recombinase, by precise reiteration of the insertion of site-specific recombination sites into the unmodified chromosome homologue and subsequent creation of a second replacement event. Such cells may therefore be used directly for in-vitro phenotypic analysis or used to make 100% ES cell-derived embryos by the tetraploid aggregation technique (Nagy A *et al* 1993).

In comparison to the teaching of WO04076618, for example as the present invention is concerned with direct replacement there is no possibility for haploinsufficiency and the integrity of the termini of the BAC replacement is assured by the replacement occurring via Cre/*lox* recombination.

### Detailed Description

The present invention will now be further described by way of example and with reference to the figures which show:

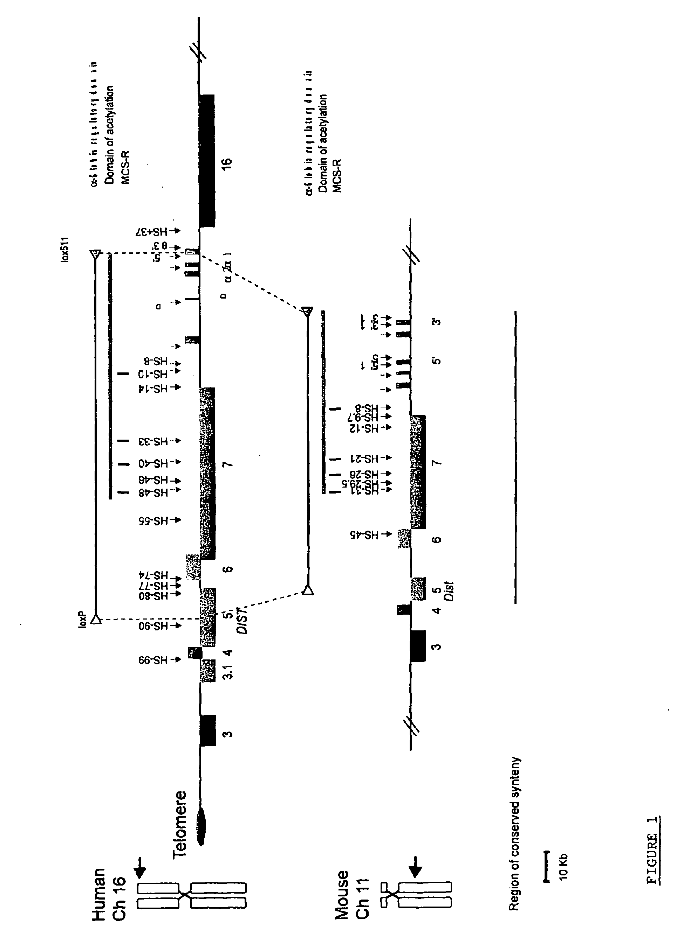

**Figure 1****. The chromosomal localization and organization of the human and mouse α globin clusters.** The human cluster (16p13.3) is located close to the telomere (oval) whereas the mouse cluster lies at an interstitial chromosomal position (11qA4). The globin genes are shown as labelled red boxes. Other human genes and their mouse orthologues are annotated as previously (Flint et al., 1997) 3=*IL9RP3,* 3.1=*POLR3K*; 4= *c16orf33*; 5=*C16orf8 (Dist)*; 6=*MPG*; 7=*C16orf35* and 16=*LUC7L* and shown as coloured boxes encoded by the top (above the line, 5' to 3' left to right) or bottom (below the line, 5' to 3' right to left) DNA strand. Note that two genes (*POLR3K* and α^{D}) are found only in the human cluster. No pseudogenes are shown apart from *IL9RP3,* which is a pseudogene in man but a functional gene in mouse. A grey line above each cluster represents the domain of acetylation that appears only in erythroid cells. MCS-R refer to multispecies conserved regulatory elements. Below this in each cluster are known erythroid-specific (red arrows) and constitutive (black arrows) DNAseI hypersensitive sites. Above this in each cluster is the extent of the α globin region exchanged between man (BAC) and mouse (ES cells) delimited upstream by the *loxP* site (yellow triangles) and downstream by the *lox511* site (purple triangles). The orientation of the *loxP and lox511* sites (as defined by their spacer sequences) is indicated by the horizontal directionality of the triangles.

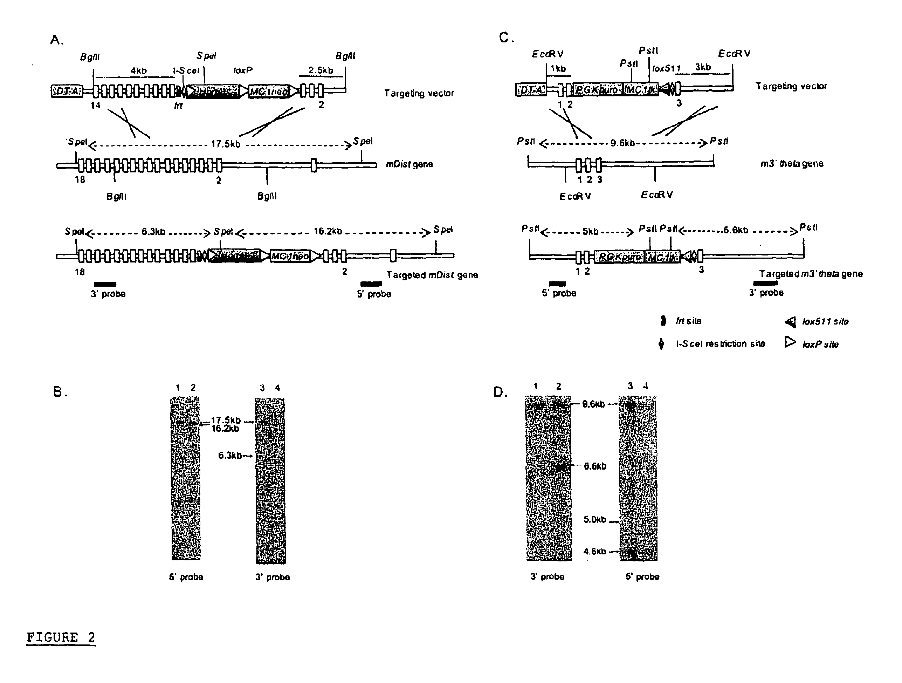

**Figure 2****. Targeting the mouse *Dist* and *3*' genes in ES cells.** (A) A chromosome engineering cassette *frt*/I*-Sce*I/*Hprt*^{*-Δ*3'}/*loxP*/MC1*neo*/*loxP* was targeted to intron 4 of the mouse *Dist* gene using a linearised replacement vector with a negative diphtheria toxin selection marker (*DT-A*) at one end to enrich for targeting events (Yagi et al., 1990). (B) Southern blot analysis of the parental ES cell line (lanes 1, 3) and a *Dist* targeted cell line (lanes 2, 4) digested with *SpeI* (0.65% gel). The normal 17.5 kb fragment is reduced to 16.2 kb when hybridised with the 5' *Dist* probe (lanes 1, 2); and to 6.3 kb when hybridised with the 3' *Dist* probe (lanes 3, 4). (C) A chromosome engineering cassette *PGKpuro*/MC1*tk*/*lox511l*I*-Sce*I was targeted to intron 2 of the mouse 3'θ gene in the *Dist* targeted cell line using a linearised replacement vector with a negative selection marker (*DT-A*) to enrich for targeting events. (D) Southern blot analysis of the parental ES cell line (lanes 1, 3) and a *Dist*/*θ* double targeted cell line (lanes 2 and 4) digested with *Pst*I (0.8% gel). The normal 9.6 kb fragment is reduced to a 6.6 kb fragment when hybridised with the 3' probe (lanes 1, 2) and to a 5.0 kb when hybridised with the 5' probe (lanes 3, 4). It should be noted that the 4.6kb restriction fragment seen with the 5' θ probe corresponds to the upstream mouse 5' θ gene sequences; all other restriction fragment sizes are derived from the 3' θ gene.
   Symbols as follows: exons - clear rectangles (some numbered); non-coding sequences and introns - narrow clear rectangles; selection markers - blue boxes; *Hprt^{-Δ3'}* segment - red box (clear indent indicates the end with the promoter and start of the 5' coding region and the blunt end the break in mini-gene intron sequence); *lox, frt* and I-*Sce*I sequences - see key, orientations indicated by horizontal directionality of triangles and chevrons; probes - black filled boxes; vector homology arm regions - dotted lines with sizes in kilobases (kb); predicted homologous recombination between vector and chromosome - crossed black lines; restriction sites - thin vertical lines; diagnostic restriction fragments - dashed lines with arrow heads, with size in kilobases (kb). For clarity the pBS component of the vectors and their site for linearization is not shown. Some components are not drawn to scale.

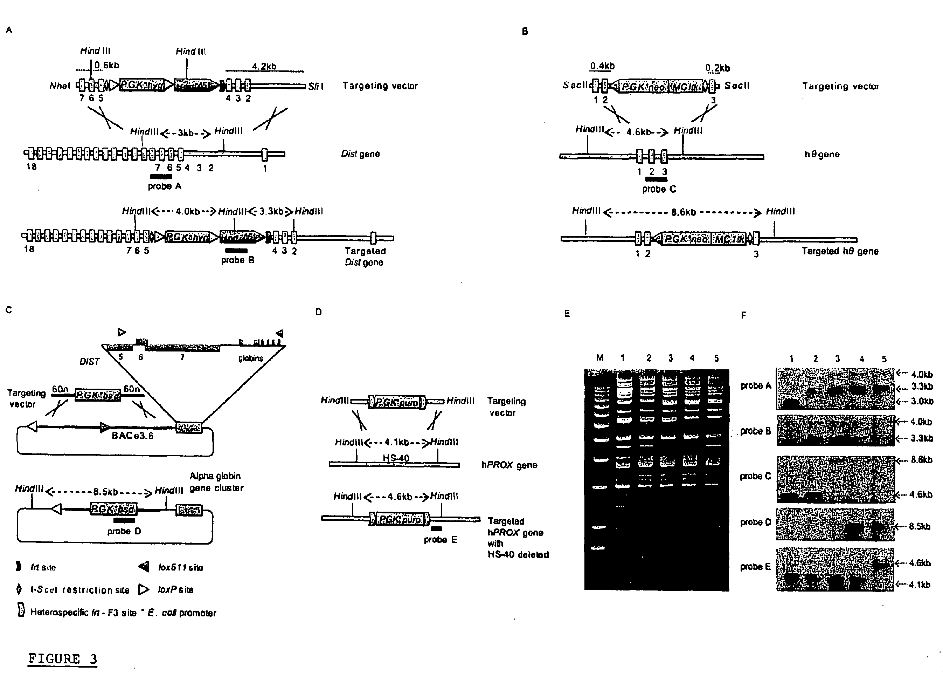

**Figure 3****. Targeting vectors used for RED recombination in *E. Coli* to insert recombination cassettes into the *DIST* and 3' gene sequences within BAC 344L6.** (A) Targeting vector used to insert a *I-Sce*I/*loxP*/PGK-*Tn905hyg*/*loxP*/*Hprt^{-Δ5'}*/*frt* cassette into intron 4 of the human *DIST* gene. (B) Targeting vector used to insert a *low511* /*PGK-Tn*905*neo*/MC1*tk*/I*-Sce*I cassette into intron 2 of the human θ gene. (C) Targeting vector with 120 nucleotides total homology used to replace the *low511* site in the BAC vector backbone with a PGK-EM7*bsd* selection cassette. The α-globin cluster contained within the BAC is shown (not to scale). (D) Targeting vector used to delete HS-40 within the *PROX gene* (C16orf35), replacing it with *a frt-* F3/PGK-EM7puro/*frt*-F3 selection cassette. (E) *Hind*III digests (0.8% gel) of BAC DNAs at the different stages of retrofitting: BAC 344L6 (lane 1), *DIST* targeted BAC (lane 2), *DIST* and human θ double targeted BAC (lane 3), triple targeted BAC in which *lox*511 site within the backbone is deleted (lane 4), triple targeted BAC with the HS-40 regulatory element deleted (lane 5). (F) Southern blot analysis of *Hind*III BAC digested DNA hybridised with probes A - E showing the expected changes in fragment sizes following each progressive modification to the BAC.
   Symbols as follows: exons - grey rectangles (some numbered); non-coding regions and introns - narrow grey rectangles; *Hprt^{-Δ5'}* segment - red box (pointed end indicates the terminus of the 3' coding region and position of the polyadenylation site, and the blunt end the break in the mini-gene intron sequence); BAC sequences - black lines, or blue lines for the 60b homologous sequences. Other symbols - as in Figure 2. The restriction enzymes used to generate the homologous ends of the targeting vectors are indicated in A, B and D; in C this was *Asc*I*.* Some components are not drawn to scale.

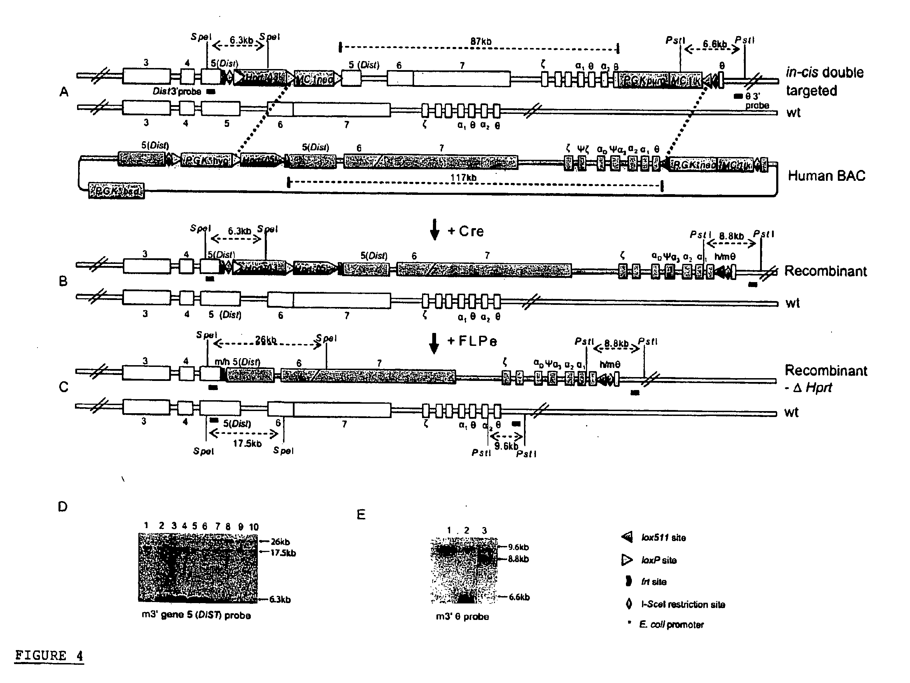

**Figure 4****.** Diagram of Cre recombination in *in-cis* or *in-trans Dist*/*θ* doubled targeted ES cells. The potential outcomes of recombination between the plasmid *pBS-loxP*/*Hprt^{-Δ5'}*/*lox511* depending on the *in-cis* or *in-trans* arrangement of *loxP and lox511* sites are illustrated. Dark blue dotted line indicates potential plasmid x chromosome or inter-chromosomal *lox x lox* interactions; light blue dotted line indicates potential intra-chromosomal *lox x lox* interactions. Symbols as in legends to Figures 2, and 3.

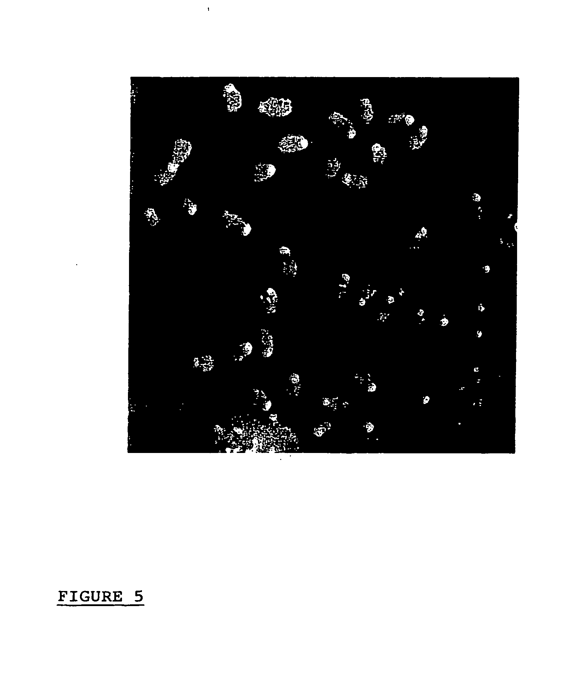

**Figure 5****. Replacement events at the mouse α globin locus.** (A) Arrangement of sequences in double targeted ES cells containing the *loxP and lox511* sites inserted *in-cis* on one chromosome, the non-targeted homologue (wt) and the modified human BAC, also showing potential Cre-mediated recombination points between modified mouse chromosome and BAC. (B) Arrangement of sequences in ES cells containing the recombinant chromosome with human sequence replacing the mouse sequence and embedded reconstructed functional *Hprt* mini-gene formed by joining of *Hprt*^{-Δ3'} and *Hprt*^{-Δ5'} segments via the *loxP* site. Non-targeted homologue is shown beneath for comparison. (C) Arrangement of sequences in ES cells containing the recombinant chromosome after removal of the *Hprt* mini-gene (- Δ *Hprt*) by FLPe recombinase. Non-targeted homologue is shown beneath for comparison. (D) Southern blot analyses following *Spe*I digestion (0.65% gel) and hybridization with mouse 3' *Dist* probe (Figure 2). Lane 1: Parental ES cells; lane 2: Double targeted cell line 2/B-6; lane 3: Double targeted cell line 2/H-1; lane 4: 2/B-6 HAT^{R} exchange clone # 1; lane 5: 2/B-6 HAT^{R} exchange clone # 2; lane 6: 2/H-1 HAT^{R} exchange clone; lanes 7 and 8: 2B-6 6-TG^{R} exchange clones; lanes 9 and 10: 2/H-1 HAT^{S} / 6-TG^{R} exchange clones. Note, lanes 4 -6 show no alteration in the *Spe* I fragment size following the exchange because the *Hprt* gene is still present. (E) Southern blot analyses following a *Pst* I digestion (0.8% gel) and hybridization with mouse 3' θ probe (Figure 2). Lane 1: parental ES cells; lane 2: Double targeted cell line 2/B-6; lane 3: 2/B-6 HAT^{R} exchange clone # 1.
   Symbols as follows: mouse genes - clear rectangles; mouse inter-genic regions - narrow clear rectangles; human genes - grey rectangles (diagonal division between 6 and 7 indicates genes overlap); human inter-genic regions - narrow grey rectangles; thin black dashed lines with bar at ends - mouse and human regions delineated by the *lox* site integrations with size in kilobases (kb); blue dashed lines - predicted *lox x lox* recombination points between BAC and chromosome to result in the exchange event. Other symbols - as in Figures 2 and 3. Diagram is not to scale.

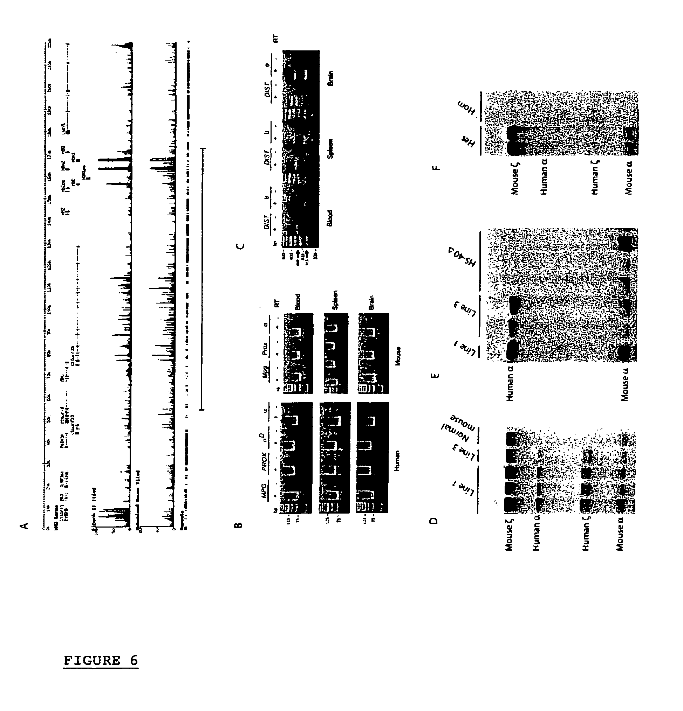

**Figure 6****. Detailed mapping of the exchanged a globin regulatory domain.** Southern blot analysis of digested DNA for humanized mice in comparison with human and mouse genomic DNA. The membranes were hybridized with probes across the entire mouse/human locus. The same size bands were observed in the BAC replacement region as in the human genomic DNA (panels B to H). Arrowheads in panels A and I indicate restriction fragments in the BAC replacement region corresponding to the correct mouse/human junctions, showing that the exchange has occurred at the end points as predicted. Molecular weight markers are shown.

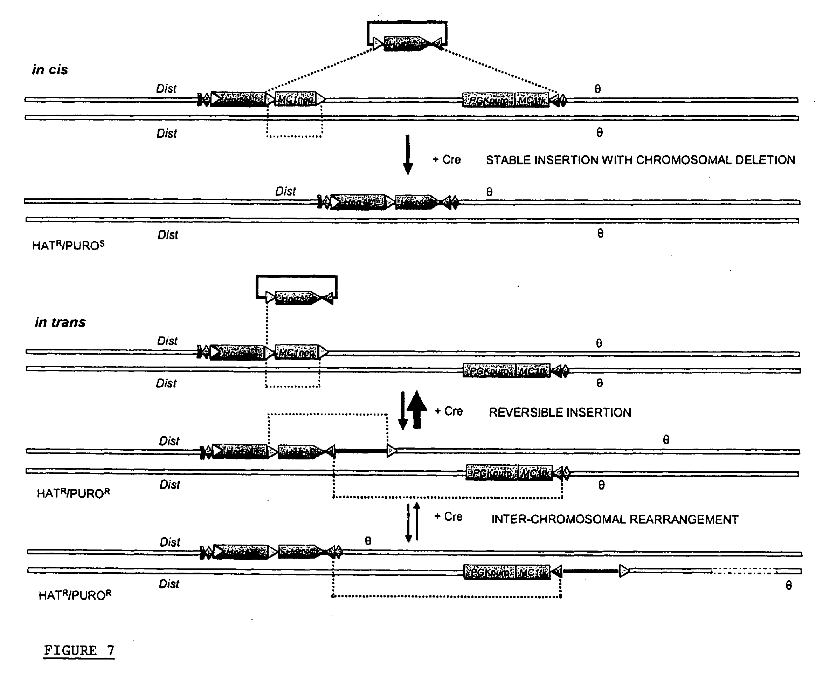

**Figure 7** **Southern blot analysis of mouse/human junctions in mice with the human BAC replacement.** Mice heterozygous for the BAC replacement after the removal of *Hprt* mini-gene (by crossing with FLPe deleter mice) were inter-crossed. Southern blot analyses of DNAs are shown from three embryos (E15) from a timed mating experiment. A. *Spel* digestion (0.65% gel) and hybridization with mouse 3' *Dist* probe; B *Pstl* digestion (0.8% gel) and hybridization with mouse 3' θ probe; C *Eco*RI digestion (0.65% gel) and hybridization with human ζ probe. Lane 1: wild type: lane 2: heterozygote and lane 3: homozygote.

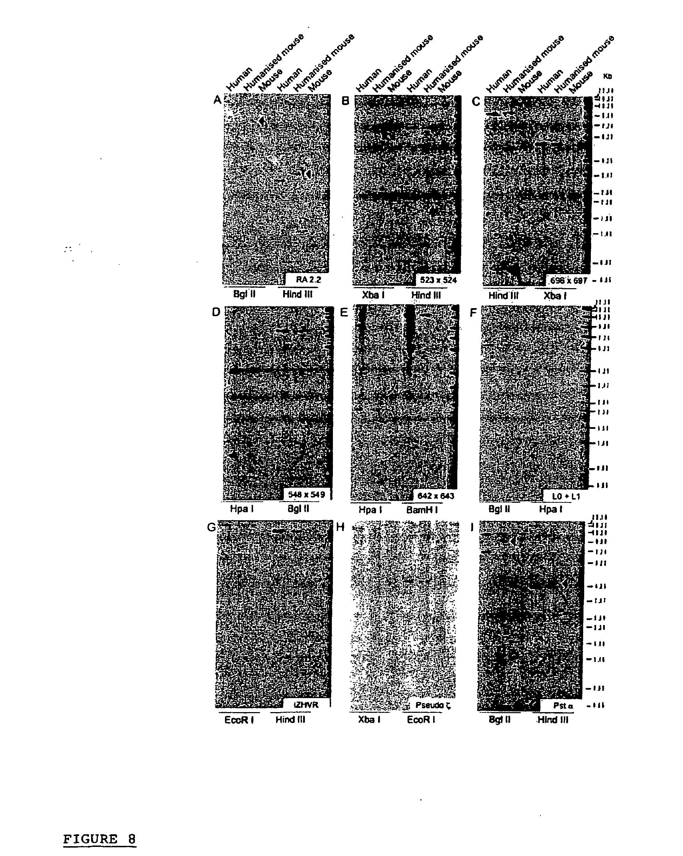

**Figure 8****. Fluorescence *in situ* hybridization** demonstrating the exchange of the human (green) and mouse (red) α globin regulatory domains within the context of mouse chromosome 11 (blue).

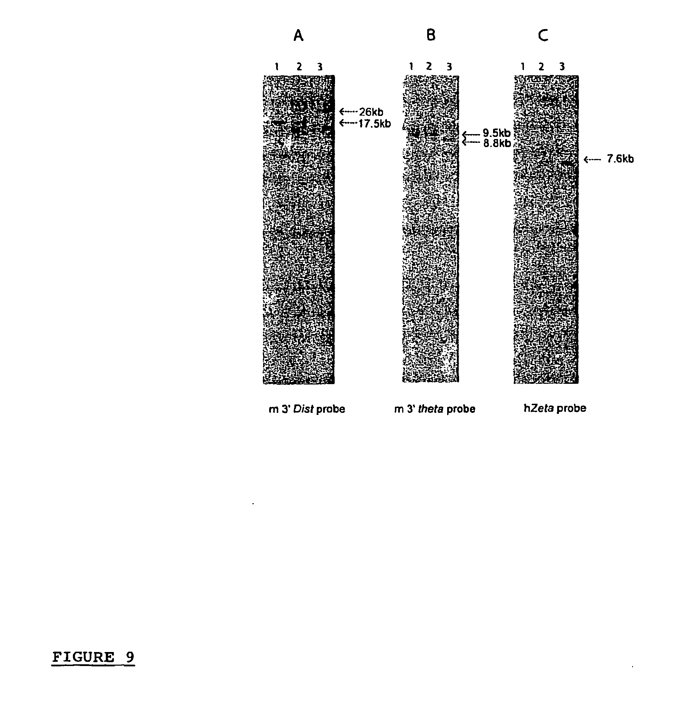

**Figure 9** **Expression from the exchanged a globin regulatory domain.** (A) Hybridization of cDNA from normal human erythroblasts (above) and erythroblasts from a mouse (line 1) homozygous for the replacement event. (B) Expression of *MPG, PROX,* α*^{D}*, and α (left panel) was detected in spleen, peripheral blood and brain samples from a heterozygote using gene-specific primers. Likewise, expression of *Mpg, Prox,* and α was present in the murine locus (right panel). Low levels of expression of α globin in non-erythroid tissues is due to contamination with small amounts of blood. (C) RT-PCR analysis of mRNA expression across the murine/human junction *(Dist* and 0 genes) in spleen, peripheral blood and brain samples of a heterozygote mouse where the *Hprt* gene has been excised from the *Dist* gene. A correct size transcript is observed across the junction (arrows) showing that the gene expression is not affected (D) RNAse protection assays of 10.5 day heterozygous embryos showing expression of mouse and human ζ and α genes (E) RNAse protection assay of adult blood RNA. (F) RNAse protection assays of embryonic blood (9.5 days) from heterozygotes and homozygotes for the recombinant chromosome from which the HS-40 element was deleted.

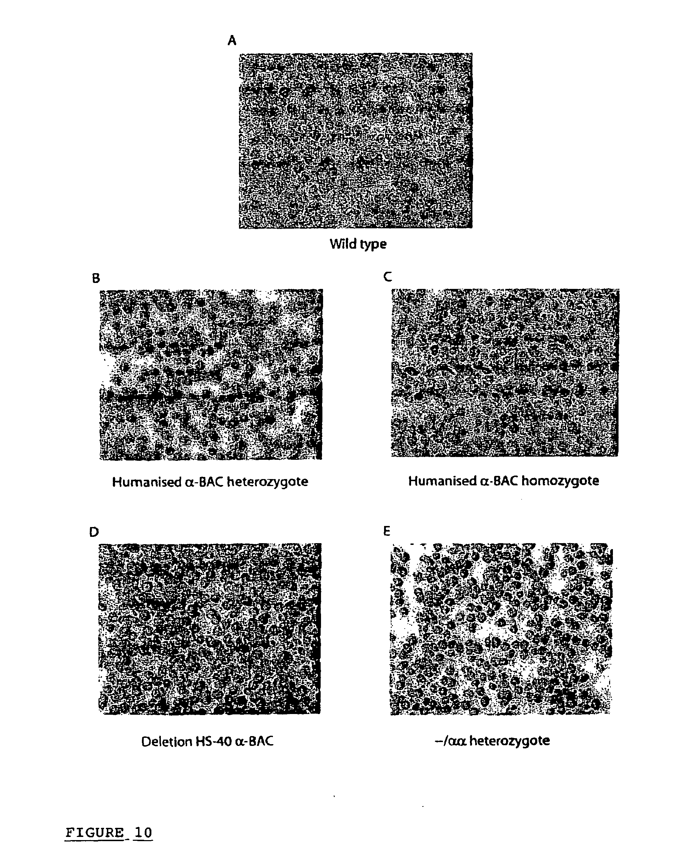

**Figure 10** **Red cell phenotypes of normal and humanized mice.** Peripheral blood films of A) wild type mouse, B) a heterozygous and C) a homozygous mouse containing the human BAC exchange. D) A heterozygote with the human BAC in which HS-40 has been deleted and E) a mouse containing two α globin gene deletions for comparison.

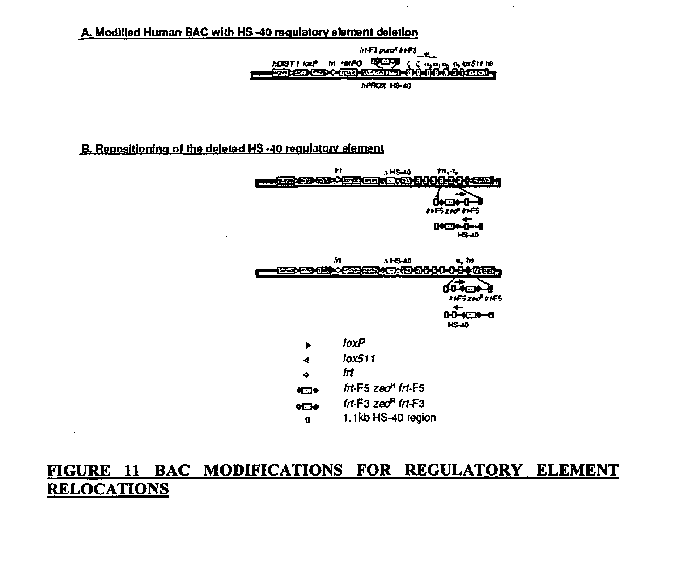

**Figure 11a** shows a schematic representation of a modified human BAC comprising an HS-40 regulatory element deletion.
**Figure 11b** shows a schematic representation of the repositioning of the deleted HS-40 regulatory element.

### Experimental Procedures

### Bacterial strains

Transformations with ligated DNA were carried out using chemical competent *E. coli* strain DH5α. The BAC 344L6 was transferred into *E. coli* strain DY380 (gift from D. Court and N. Copeland) selected in chloramphenicol 12.5µg/ml and electrocompetent cells then prepared for retrofitting (Lee et al., 2001).

### ES cell gene targeting vectors

Replacement vectors were designed for positive-negative selection and constructed in pBluescript (pBS) plasmid by standard methods using 129/Ola cloned genomic DNA for homology arms.

m*Dist* vector:- A 6.5kb *Bgl*II restriction fragment encompassing intron 4 was recovered from a 129/Ola genomic library and inserted into the *Bam*HI site of pBluescript (pBS) to provide the homology arms for the vector. A chromosome engineering cassette was constructed comprising, in order, the following linked components: a *frt* site; an *Isce*I restriction site; a defective segment of a human *Hprt* minigene (*Hprt*^{-Δ5'} - Smith *et al* 1995); a *loxP* sequence; a neomycin resistance marker (PGK*neo*); and a *loxP* sequence (in the same orientation as the first *loxP* site). This cassette was inserted at an *Avr*II site in intron 4 of the mouse *Dist* sequence leaving 2.5kb and 4kb of homology at the 5' and 3' ends of the vector respectively. A 2.9kb *Xho*I *- Sal*I fragment containing a Diphtheria toxin-A chain (*DT-A*) cDNA linked to a CAGGS expression cassette (Priddle, H. and Smith, A.J.H. unpublished) was inserted at the end of one of the homology arms at the *Sal*I site within the polylinker of pBluescript (pBS) to provide a negative marker for targeting (Yagi et al., 1990). The targeting vector was linearized with *Not*I prior to electroporation.

m 3'θ vector: A 4.3kb *Eco*RV restriction fragment encompassing intron 2 from a 129/Ola genomic library was inserted into the *Eco*RV site in pBS to provide the homology arms for the vector. A chromosome engineering cassette was constructed comprising, in order, the following linked components: a puromycin resistance selection marker (PGK*puro*); a negative selection marker MC1*tk* (Mansour et al., 1988); a *lox511* sequence; and an *I-Sce*I restriction site. This cassette was inserted at a *Sma* I site in intron 2 of the 3'θ gene leaving 1kb and 3.2kb of homology at the 5' and 3' ends of vector respectively. A 2.9kb *Xho*I - *Sal*I fragment containing a *DT-A* marker (as above) was inserted at the end of one homology arm at a *Not*I blunted site within the polylinker of pBS. The vector was linearized at the *Sal*I site in the pBS backbone prior to electroporation.

### BAC retrofitting vectors

Vectors were constructed in pBS plasmid by standard methods using subcloned human BAC 344L6 DNA or duplex synthetic oligonucleotides for homology arms, and selection cassettes with dual eukaryotic (PGK) and prokaryotic (Tn903 or EM7) promoters (Testa et al., 2003).

h*DIST* vector: A *Pml*I (blunted) - *Bam*HI 6.9kb fragment encompassing intron 4 from the human BAC 344L6 was inserted into a modified pBS (*Sfi*I site replacing the *Not*I site) cut with *Kpn*I and *Bam*HI (blunted) to provide the homology arms for the vector. A chromosome engineering cassette was constructed comprising, in order, the following linked components: an *I-Sce*I site; a *loxP* site; a hygromycin selection marker with both bacterial and mammalian cell promoters (PGK-Tn903*hyg*); a *loxP* sequence; a defective segment of a human *Hprt* minigene (*Hprt* ^{*-Δ*3'}) (Smith et al., 1995); *and a frt site.* This cassette was inserted into a *Kpn* I site within intron 4 of the *DIST* gene. The targeting fragment for RED recombination was excised from pBS with *Nhe*I plus *Sfi*I*.*

hθ:- A 0.6kb *Sac*II fragment encompassing intron 2 of the θ gene from the human BAC 344L6 was inserted into the *Sac*II site of pBS. A chromosome engineering cassette was constructed comprising, in order, the following linked components: a neomycin resistance marker with both bacterial and mammalian cell promoters (PGK-Tn903*neo*); a *lox511* sequence; a MC1*tk* marker; and an I-*SceI* site. The above cassette was inserted into a *Sma* I site within intron 2 of the hθ gene. The targeting fragment for RED recombination was excised from pBS with *Sac* II.

Removal of *lox511* from the BAC backbone: the BACe3.6 contains both a *lox511* and a *loxP* sequence in the backbone. To remove the *lox511* sequence a vector with 60 nucleotide long homology arms flanking the *low511* site was constructed. This was made by first annealing complementary oligonucleotides corresponding to the sequence across the 120 nucleotide region designed with a unique restriction site in the centre. The oligonucleotides were also designed to create duplex DNA with *Asc*I restriction site compatible ends and were subsequently inserted into a modified pBS vector with a unique *Asc*I site. A blasticidin resistance marker (*bsd*) with both bacterial and mammalian cell promoters (PGK-EM7*bsd*) was inserted into the central restriction site in the oligonucleotide duplex sequence. The targeting fragment for RED recombination was excised with *Asc*I.

HS-40 deletion:- A 4.1kb *Hind* III fragment encompassing the HS-40 region from the human BAC 344L6 was inserted into pBS. A 1.1kb *Hpa*I fragment which spanned the HS-40 regulatory element was excised and replaced by a puromycin resistance marker with both bacterial and mammalian cell promoters (PGK-EM7*puro*). This marker was flanked by a pair of *frt-F3* sites (Schlake and Bode, 1994). The targeting fragment was excised for RED recombination with *Hind*III.DNA restriction fragments used for electroporation were purified from the pBS backbone by gel electrophoresis.

### BAC modifications

The RED recombination protocol (Lee et al., 2001) was carried out by electroporating 50µl of the cells with 150ng - 300ng purified construct in a 0.1cm wide cuvette using a Bio-rad gene pulser set at 1.8kV. Immediately after the electroporation, 1 ml of SOC media was added and the cells were further grown at 32°C for two hours before being plated on appropriate selective agar media. Drug selection concentrations were as follows: puromycin - 62.5 µg/ml; kanamycin - 12.5 µg/ml; blasticydin - 100 µg/ml; hygromycin - 50µg/ml.

### ES cell culture and transfection conditions, drug selection, and identification of targeted clones

ES cells were grown without feeder cells throughout on gelatin in LIF-supplemented GMEM with serum (Nichols et al., 1990). 75µg of *Not*I linearized *Dist* targeting vector was electroporated into 5 x 107 E 14 TG2a.IV cells as previously described (Smith et al., 2002). Cells were selected in 160µg/ml G418 and resistant colonies picked after eight to ten days into 96 well plates, and subsequently replica 96 well plates made for clone freezing and DNA analysis. Targeted clones were identified by Southern blot analysis of *Spe* I digested clone DNAs and hybridization with probes external to the homology at the 5' and 3' ends (a 0.96kb *BamH*I *- Hind*III fragment for the 5' probe and a 0.90kb *Spe*I *- Bgl*II fragment for the 3' probe). The *Dist* targeted cell line was then used for a second round of transfection to target the 3' θ gene according to the procedure above. Cells were selected in 1.5µg/ml puromycin. Targeted clones were identified by Southern blot analysis of *Pst*I digested DNA and hybridized with probes external to the region of homology (a 0.22kb *Xho* I/*Hind* III fragment for the 5' probe and a 0.45kb *Bgl*II fragment for the 3' probe).

### Determination of in-cis double targeted clones

Double targeted ES cell clones were co-electroporated with 75µg supercoiled pBSbased vector containing a *loxP*/*Hprt^{-Δ3'}*/*lox511* cassette and 25µg supercoiled pCAGGS-Cre-IRES*puro* plasmid (Smith et al., 2002). 24 hours post-electroporation the cells were trypsinized, counted and plated at the density of 5 x 106 cells per plate. Cells were selected the following day in HAT supplemented medium (0.1 mM hypoxathine, 0.4µM aminopterin, 0.016mM thymidine). After 10 days HAT^{R} colonies were counted, picked into 96 well plates and subsequently replica 96 well plates made for G418 and puromycin resistance/sensitivity testing. Details are provided in S3.

### BAC exchange

Approximately 80µg of retrofitted supercoiled BAC DNA and 30µg supercoiled pCAGGS-Cre-IRES*puro* plasmid DNA (approximate 1:10 molar ratio) were coelectroporated into 5 x 10⁷ - 1 x 10⁸ double targeted ES cells (0.4cm Bio-rad cuvette, 0.25kV, 960µF). Twenty four hours later HAT supplemented media was added and left for four days. The medium was then changed to HT (0.1 mM hypoxanthine plus 0.016mM thymidine) supplemented media and resistant colonies were picked six to ten days later into a 96 well plate. The clones were subsequently expanded for freezing, for DNA analysis and for testing resistance/sensitivity to the following drugs: G418 (160µg/ml); hygromycin (120µg/ml); puromycin (1.5µg/ml); blasticydin (5 µg/ml); Ganciclovir (2.5µM); 6-thioguanine (10µM); HAT (0.1mM hypoxanthine, 0.4µM aminopterin, 0.016mM thymidine). DNA of potential replacement clones was first analysed at the end points of the exchange event by *SpeI* digestion and hybridization with 5' and 3' mouse *Distl* probes, and *Pst*I digestion and hybridization with 5' and 3' mouse θ probes.

### Removal of the Hprt cassette

Clones with a confirmed exchange event were electroporated with 75µg supercoiled pCAGGS-FLPe-IRES*puro* plasmid DNA (Schaft et al., 2001). The cells were grown non-selectively for six days and then plated at a density of 10³ per 10cm plate in medium with 10µM 6-thioguanine. Resistant colonies were picked after eight days into a 96 well plate, and subsequently replica 96 well plates made for freezing and DNA analysis.

### Generation of Chimeras and breeding

All animal experiments were conducted under Home Office licences after ethical review of the Universities of Edinburgh and Oxford. Exchanged ES cells were injected into C57/BL6 blastocysts and transferred to a pseudopregnant female mouse. Male chimeric pups were test-crossed with C57/BL6 female mice to test for germline transmission. DNA from agouti F1 test-cross offspring was genotyped by *Eco*RI digestion of genomic DNA and hybridization to a probe specific to human ζ gene. F1 test-cross positive heterozygotes were inter-crossed to derive homozygotes, and back-crossed onto the C57BL/6 strain to maintain the lines. F1 test-cross positive heterozygotes were also crossed with a C57BL/6 FLPe germ line deleter line to derive lines with the *Hprt* cassette removed, and these subsequently maintained by back-crossing onto the C57BL/6 background and inter-crossed to derive homozygotes.

### DNA and RNA analysis

Genomic DNA analysis and RNAse protection assays were as previously described (Anguita et al., 2002). DNA FISH studies were as described (Brown et al., 2006).

### Microarray analysis and RT-PCR

Total RNA was isolated from adult tissues or whole embryos using the Trizol reagent (Sigma). RNA was treated with Ambion's DNA-free^{™} Kit and complementary DNA and control reactions, without enzyme, was synthesized using StrataScript® First-Strand Synthesis System, according to the manufacturer's instructions. The Affymetrix array was analysed as previously described (De Gobbi et al., 2006). Quantitative Real-time RT-PCR of the first-strand cDNA was performed (ABI Prism 7000 Sequence Detection System) to measure the expression of the globin genes. Expression of the human α and mouse α genes was normalized to the expression of mouse α globin. Expression of other genes in the locus (*MPG, PROX* and α^{D}) was measured qualitatively by Real-time RT-PCR and run on polyacrylamide gels stained with ethidium bromide. Specific primers and TaqMan probes were designed with Primer Express software (ABI) and sequences used are provided in Supplementary Table 2.

RT-PCR of the first-strand cDNA across the mouse/human junctions (*Dist* and θ genes) was performed with Herculase Hot Start PCR (Stratagene) and with the following set of primers: *Dist* junction forward and reverse, θ junction forward and reverse (Supplementary Table 2). Products were run on agarose gel and stained with ethidium bromide.

### Genomic replacement strategy for substitution of the α globin gene domain

We set out to exchange an extensive part of the mouse genome with its human equivalent in a region where the consequences could be clearly analyzed. We chose the region encompassing the α globin gene cluster since this has been fully characterised both structurally (Flint et al., 2001; Hughes et al., 2005; Kielman et al., 1996) and functionally (Anguita et al., 2004; Anguita et al., 2001; Anguita et al., 2002) in both mouse and man. The gene order and transcriptional orientation of the globin genes and other widely expressed genes flanking them in this segment of the genome are highly conserved (Figure 1); a multispecies DNA comparison shows that the region of most conserved synteny extends from the *DIST* gene (a rhomboid-like gene also known as c16orf8, gene 5 in Fig 1) to the θ globin gene (Hughes et al., 2005). This region contains all known multi-species conserved regulatory elements (MCS-Rs), the corresponding erythroid-specific DNase1 hypersensitive sites and well defined segments of chromatin which become acetylated specifically in erythroid cells when the human and mouse α globin genes are expressed (Anguita et al., 2001; Anguita et al., 2002; Higgs et al., 1998). This region is therefore thought to encompass the entire α globin regulatory region (Figure 1).

In outline, the plan involved insertion of non-interacting (heterospecific) *lox* recombination sites (*loxP* and *low511*) into positions (in the *Dist* and θ genes respectively) flanking the α globin regulatory domain in the mouse ES cell chromosome, and in the corresponding regions of a BAC (344L6) that spans the entire (~120kb) human α globin region. In the presence of Cre recombinase, reciprocal exchange of sequence between the BAC and mouse chromosome should occur since only the compatible *loxP*/*loxP* and *lox511*/*lox511* sites will recombine (Bethke and Sauer, 1997) and thus result in replacement of the entire mouse regulatory region in the ES cell genome with the corresponding human region (Figure 1).

We chose the fourth intron of the *DIST* gene in both mouse (ES cells) and human (BAC) sequences for insertion of the *loxP* site (upstream exchange points); the second intron of the 3' θ gene in mouse (ES cells) and the corresponding region of the second intron of the single human 9 gene (BAC) (Figures 1-3) was chosen for insertion of the *lox511* site (downstream exchange points). These positions were chosen to avoid inadvertently disrupting any regulatory elements (MCS-R in Figure 1), RNA splicing or the open reading frame. When recombination is activated this should result in the exchange of 117kb of human sequence for the equivalent 85kb of mouse sequence, creating a hybrid 5'mouse-3'human *DIST gene* upstream and a hybrid 5'human-3'mouse θ gene downstream in the recombinant mouse chromosome.

### Targeted modifications of the a globin regulatory domain in mouse ES cells

The strategy required the sequential insertion of a *loxP* site and then a *lox511* site (in inverted orientation) into the upstream and downstream positions of the mouse α globin cluster by conventional homologous recombination (Mansour et al., 1988). The vectors designed to achieve this also integrated genetic markers directly linked to the *loxP* and *lox511* sites to enable selection of the chromosome replacement in the second stage. Positive genetic selection for *loxP* x *loxP* recombination involved reconstruction of a functional hypoxanthinephosphoribosyltransferase (*Hprt*) minigene from defective 5' and 3' components (Smith et al., 1995), and negative selection for *lox511* x *lox511* recombination involved the use of HSV thymidine kinase (*tk)* genes.

For the mouse *Dist* gene a targeting vector containing a *frt*/*I-SceI*/*Hprf* ^{-Δ3'}/*loxPl*MC1*neo*/*loxP* cassette (Fig. 2A) was transfected into HPRT deficient (HPRT-) E14-TG2a.IV ES cells and correctly targeted clones were obtained (Fig. 2B). One of these *Dist* targeted clones was used for a second round of homologous recombination to target the θ gene using a vector with the *PGKpuro*/MC1*tk*/*lox511*/*Isce*I cassette (Fig. 2C) and several independently targeted clones were obtained. Double *Dist-*θ targeted clones should contain the integrated cassettes either on the same chromosome (in-*cis* targeting) or on each of the two homologues (in-*trans* targeting). We distinguished these based on the efficiency of Cre-mediated recombination when a plasmid *pBS-loxP*/*Hprt^{Δ5'}*/*lox511,* co-transfected with a Cre expression plasmid, interacted with the integrated cassettes reconstructing a functional *Hprt* mini-gene. Cells with sites *in-cis* should result in a higher frequency of recombination relative to those *in-trans,* as measured by the frequency of HPRT⁺ colonies. The reason for this is best explained by reference to Figure 4. *In-cis* targeted clones permit recombination with the incoming plasmid via both *loxP* x *loxP* and *low511* x *low511* recombination events (either simultaneously or sequentially) reconstructing an *Hprt* mini-gene in a stable structure in which the newly integrated plasmid component cannot be excised by further Cre recombination (due to *loxP* x *lox511* incompatibility), i.e. it is 'locked in'. *In trans*-targeted-clones can reconstruct the *Hprt* minigene by *loxP* x *loxP* recombination but in this case the additional *lox511* x *lox511* recombination event cannot occur since the chromosomal *lox511* site is on the homologue. This reconstructed *Hprt* mini-gene is thus in an unstable arrangement since a pair of *loxP* sites remain present so the entire newly integrated plasmid can be immediately excised in the continued presence of Cre recombinase activity by precise reversal of the *loxP* x *loxP* recombination event, which is an efficient intra-chromosomal event (denoted by the anti-parallel arrows with the efficient reverse reaction indicated by the bolder arrow). In the *in-trans* situation *lox511* x *lox511* recombination could, in principle, occur by interchromosomal recombination, which is shown in the Figure. However, inter chromosomal Cre recombination is two to three orders of magnitude less efficient (Zheng et al., 2000) so this is unlikely to contribute to stabilization of the reconstructed *Hprt* mini-gene. On this basis, three from twelve independent, cell lines (2B-6, 2/H-1 and 2/H-10) were assigned as having *in-cis* targeted *Dist* and 9 genes (see Table 1).

### Targeted modifications of the human α globin cluster in a BAC

A human BAC clone, 344L6, covering the interval from 3kb upstream of the *IL-9RP3* pseudogene to 18kb downstream of the θ gene provided the human sequences. This BAC was modified by inserting *loxP* and *lox511* sites (in the equivalent positions and in the same orientations as in the mouse chromosome) using RED-mediated homologous recombination in the *E.coli* strain DY380 (Lee et al., 2001). The human *DIST* gene was targeted with the vector containing the I-*SceI*/*loxP*/*PGKTn903hyg*/*loxP*/*Hprt^{Δ5'}*/*frt* cassette (Figure 3A) and the θ gene was targeted with the vector containing the *lox511*/*PGK-Tn903neo*/MC1*tk*/I-SceI cassette (Figure 3B). Finally, it was necessary to remove a second, pre-existing, endogenous *lox511* site present in the BACe3.6 vector; an additional recombination event replaced the endogenous *lox511* site with a blasticidin selection cassette (Figure 3C).

### Selection and characterization of the genome replacement event

The vectors used to target the mouse and human *DIST* genes (Figures 2A and 3A) inserted the *loxP* sites and their linked *Hprt^{-A3'}* and *Hprt^{-Δ5'}* sequences respectively in the opposite transcriptional orientation to the *Dist* gene. This should create a reconstructed *Hprt* mini-gene after Cre-mediated recombination located in the recombinant mouse chromosome. The vectors used to target the mouse and human θ genes (Figures 2B and 3B) inserted the *lox511* sites in the same orientation but opposite to that of the *loxP* site. The relative order of the *lox511* and linked HSV thymidine kinase negative selection marker, MCl*tk*, should, after Cre-mediated recombination, result in a recombinant mouse chromosome lacking a negative selection marker.

This overall arrangement of targeted sequences in the ES cells and the BAC necessary to implement the desired Cre-mediated exchange is illustrated in Figures 5A and 5B. The reciprocal recombinant BAC product (not shown) should remain unintegrated and therefore be lost after cell division. ES cells with the resulting recombinant mouse chromosome will have an HPRT⁺, ganciclovir resistant phenotype.

Two cell lines, 2/B-6 and 2/H-1, with putative *in-cis* targeted events at the *Dist* and θ loci were co-transfected in an approximate 1:10 molar ratio with the modified BAC and a Cre recombinase expressing plasmid (pCAGGS-Cre-IRES*puro*) (Smith et al., 2002), and HPRT⁺ cells subsequently selected in medium with hypoxanthine, aminopterin and thymidine (HAT). HAT resistant (HAT^{R}) colonies were obtained at an approximate frequency of 1x10⁻⁸. Two independent colonies derived from the 2/B-6 ES cell line and one from the 2/H-1 line were obtained. All three were resistant to ganciclovir indicating that Cre mediated exchange had also occurred via the *lox511* site at the θ gene (Figures 5A and B). In addition, the cells were sensitive to hygromycin, puromycin, and G418 indicating that the expected loss of these markers had occurred (Figure 5B). Sensitivity to blasticidin suggested that there was no random integration of the reciprocal BAC product of the exchange event nor had the BAC DNA integrated by non-homologous recombination.

The reconstructed *Hprt* mini-gene embedded in the *Dist* gene and now flanked by directly repeated *frt* sites was subsequently excised by electroporating the Flpe expressing plasmid pCAGGS-Flpe-IRES*puro* (Schaft et al., 2001) into the cells. Resistant clones were recovered at a high frequency after selection in media supplemented with 6-thioguanine (6-TG). This final modification resulted in a replacement event in which the only extraneous sequence remaining is an *frt* site at the *Dist* end of the interval and a *lox511* site and I-*Sce*I site at the 9 end (Figure 5C).

To confirm the anticipated Cre and Flpe recombination events had occurred at the exchange end-points, genomic DNA from the HAT^{R} and 6-TG^{R} clones was analysed by extensive Southern blot analysis. Fragments of the predicted sizes (Figures 5D and E, Figure 6, and Figure 7) confirmed that the expected mouse-human junction sequences had been created at both ends of the exchange interval.

### In vitro analysis of globin gene expression in ES cells

α globin expression was analysed in ES cells in which the mouse α globin locus had been exchanged for the human after *in vitro* differentiation along the erythroid lineage (Wiles and Keller, 1991). HATR ES cells with the BAC replacement (derived from the 2/B6 cell line) and control E14-TG2a.IV cells were differentiated *in vitro* for 8 days as previously described (Tufarelli et al., 2003). The resulting embryoid bodies were stained with benzidine to detect hemoglobin and total RNA was extracted to perform quantitative Real-Time RT-PCR. The results showed that the expression of human α globin was present at approximately 69% of the murine α globin gene (data not shown).

### Generating mice containing the human α globin gene cluster replacement.

The HAT^{R} cell lines with replacements containing the wild type human α globin gene cluster (2/B-6R1, 2/B-6R2 and 2/H-1R1, all of which retained the *Hprt* mini-gene) were used to generate chimeras. All three ES cell lines gave rise to male chimeras which transmitted the recombinant chromosome.

Of the three mouse lines in which the human α globin regulatory domain replaced the mouse domain, one (line 1, derived from 2/B-6R1) was mapped in detail and a second (line 3, derived from 2/H-1R1) in less fine detail. In both lines the α globin regulatory domain was shown to be intact and structurally normal (Figure 6). Line 2 (derived from 2/B-6R2) showed some anomalous bands and further investigation (data not shown) demonstrated this line had an internal deletion of the BAC replacement region that encompassed at least some of the *PROX* gene sequence but did not extend into the sequence of the *MPG* or ζ genes. This deletion was subsequently found to be present in the ES cell line and presumably arose during the exchange event. This line was not analysed further.

When heterozygotes on a mixed 129/B16/CBA genetic background from both lines (1 and 3) were mated, progeny homozygous for the recombinant chromosome were produced but not in the expected 1 in 4 Mendelian ratio (3+/+:20+/-:12-/-). Surviving homozygotes developed normally and were fertile. However all homozygotes had significant haematological abnormalities (see below). From homozygote x heterozygote crosses 6/16 homozygotes were obtained. Some homozygotes appeared, therefore, to be lost *in utero* and of those that were born, 3/9 died in the neonatal period.

Mice from lines 1 and 3 were crossed with a *Flpe* recombinase germ line deleter C57B1/6 strain (unpublished). Analyses of mice carrying the recombinant chromosome and the *Flpe* transgene confirmed deletion of the *Hprt* gene as predicted and these were subsequently inter-crossed. Southern blot analysis identified embryos and pups from the inter-cross that were homozygous for the now modified recombinant chromosome (Figure 7). Two viable homozygotes lacking the *Hprt* gene were produced and were haematologically indistinguishable from those that retained the *Hprt* gene demonstrating that the presence of the *Hprt* gene and its regulatory elements had no apparent effects on the expression of genes within the α globin region (Table 2). On a C57B1/6 genetic background, third generation heterozygotes when inter-crossed produced a similar deficiency of homozygous relative to heterozygous progeny as evidenced above.

### In vivo analysis of mice carrying a human α globin gene cluster replacement.

To visualise the exchange, splenic lymphocyte metaphases from a heterozygote with the recombinant chromosome (line 1) were hybridized with differentially labeled probes for the mouse and human α globin gene clusters together with a chromosome 11 paint. In each cell, one chromosome 11 carried a human hybridization signal while its homologue bore a mouse signal, confirming that one of the mouse chromosomes had the human replacement event and that no additional insertions had occurred (Figure 8).

Expression of the genes from the region containing the human α globin domain was first checked by hybridization of RNA from the erythroblasts of a homozygous, humanised mouse to an Affymetrix tiled microarray containing all unique sequences in the terminal 300kb of chromosome 16 (De Gobbi et al., 2006), including the entire α globin exchange region. The pattern of expression was very similar to that seen from normal human erythroblasts (Figure 9A). Using RT-PCR, transcripts from the *MPG, PROX,* α^{D} and α^{A} genes were readily detected in peripheral blood and spleen (Figure 9B). The low levels of α globin the *DIST* gene breakpoint was detected using 5' mouse and 3' human primers in brain, spleen and blood from a heterozygote from which the *Hprt* mini-gene had been excised. Similarly we detected the hybrid RNA crossing the 3' breakpoint which lies in the human and mouse θ gene (Figure 9C).

Expression of human and mouse α globin mRNA was measured in adult peripheral blood samples by both quantitative real time PCR and by RNase protection assays (RPA). The results (Table 2 and Figures 9D-F) were similar for both lines analysed and by both methods and show that expression from the recombinant chromosome is about 40% that of the mouse chromosome. At 9.5d gestation, embryos expressed high levels of the embryonic ζ globin gene (ζ^{H}/(α + ζ)^{H} ~70%, ζ^{H}/ζ^{M} ~60%); the absence of ζ gene expression in adults (Figure 9D) confirms that the normal developmental pattern of globin gene expression is retained.

Blood films from the mice carrying one copy of the recombinant chromosome were largely normal in appearance with a minor increase in anisopoikilocytosis (abnormally shaped cells). No difference was evident between the two lines. Red cells from the homozygotes were more markedly abnormal, with microcytic (small), hypochromic (pale) anisopoikilocytotic cells producing a typical thalassemic picture (Figure 10). Blood counts from an automated counter (ABX Pentra 60) confirmed that in heterozygotes the Hb level, mean cell volume (MCV) and mean cell hemoglobin (MCH) were normal while the homozygotes had normal Hb levels but a raised reticulocyte count and reduced MCV and MCH values.

### Deletion of the HS-40 regulatory element in the α-globin gene cluster.

The RMGR strategy was designed to facilitate rapid recursive introduction of mutations into the exchange region by additional rounds of BAC modification using RED-mediated recombination prior to Cre recombination in ES cells. Creating such 'in context' mutations would, for example, allow systematic *in vivo* functional analyses of human regulatory elements. As a 'proof of principle', *RED*-mediated recombination was used to remove a 1.1kb region containing the HS-40 regulatory site which lies in intron 5 of the C160rf35 (also known as the *PROX* gene; gene 7 in Figure 1) gene (Figure 3D) in the triple targeted BAC. This was accomplished using a vector in which the 1.1kb region was replaced with a PGK/EM7*puro* selection cassette flanked with direct repeats of the heterospecific *frt* site F3 (Schlake and Bode, 1994); the *frt-F3* sites were used to enable its subsequent excision by FLPe recombinase without intra-chromosomal recombination occurring with the *frt* sites flanking the *Hprt* mini-gene. Figures 3D and F (lane 5) show the expected change of a *Hind*III fragment from 4.1 kb to 4.6 kb. The modified BAC was co-electroporated into the recipient 2/B-6 ES cells, as described above, in the presence of Cre, and cells were selected in HAT medium. A single HAT resistant clone, resistant to ganciclovir and puromycin, was recovered, consistent with the anticipated replacement recombination event.

### In vitro analysis of globin gene expression in ES cells with the BAC replacement containing the HS-40 deletion.

HAT^{R} ES cells with the BAC replacement in which HS-40 had been replaced by a puromycin gene flanked by *frt-F3* sites were transfected with the FLPe expression vector and selected in 6-TG to remove both the PGK-EM7puro cassette at the deletion position and the reconstructed *Hprt* selection marker. 50% of the 6-TG^{R} clones screened were identified as being puromycin sensitive indicating efficient codeletion. Further analysis by Southern blotting showed that there was no recombination between the *frt-F3* sites and the *frt* sites flanking the reconstructed *Hprt* cassette. These ES cells were then differentiated *in vitro* as above and total RNA was prepared for quantitative Real-Time RT-PCR. The expression of the human α globin genes were shown to be <1% of the mouse α globin genes. In addition the expression of the embryonic ζ globin was detected at around 25% of the mouse counterpart. The results from the *in vitro* analysis were consistent with the results *in vivo* (see below), showing that the HS-40 regulatory element is the major regulatory element of the human α globin gene cluster.

### Characterization of mice carrying a human α globin gene cluster replacement with the HS-40 deletion.

The HATR cell line containing the human α-globin gene cluster from which HS-40 had been deleted (but retaining the *Hprt* mini-gene) also generated a transmitting male chimera. Mapping of this line showed only the expected band sizes.

When these heterozygotes were crossed, homozygotes were phenotypically indistinguishable from littermates at 10.5d gestation but by genotyping were detectable at normal frequencies. By 11.5d gestation, homozygotes were identifiable as small, pale dead embryos that had presumably died from severe anemia.

Expression of the human α globin genes in adult heterozygotes carrying the human cluster from which the major regulatory element (HS -40) had been deleted (Figure 9E) was reduced to barely detectable levels (< 2%), in keeping with results from human patients in whom this element is missing (Higgs et al., 1998). In embryos, human RNA levels were again severely reduced with ζ not as extremely affected as α (Figure 9F).

Red cell morphology was also abnormal in mice carrying the mutated BAC (ΔHS - 40); hypochromia, microcytosis and target cells were prevalent as well as marked anisopoikilocytosis (Figure 10). These films were similar to those from thalassemic mice heterozygous for a targeted deletion (Pászty et al., 1995) of both α globin structural genes (--/αα). Blood counts showed a reduced Hb level and low MCV and MCH, similar to those from --/αα mice (Table 2). The puromycin selection marker gene, which replaced the HS-40 element, and the *Hprt* mini-gene were excised from the BAC replacement by crossing this line with the Flpe recombinase deleter line. Haematological analyses of two adult mice derived from this cross carrying the now modified, mutated BAC were indistinguishable from mice in which these selection marker genes had not been excised (Table 2).

### Introduction of HS-40 element from the human α globin genre cluster into a different location

The inventors have further applied the RMGR strategy to try to define the parameters of the major regulatory element (HS -40) of the α-globin domain. This fully exploits the inherent advantage of the RMGR strategy (recursive selectable replacement with variously modified BACs) and it is argued establishes a paradigm for how complex human regulatory mutations in any genomic region can be systematically introduced and studied in ice.

Using the ability to successively modify BACs by *red* homologous recombination in *E.coli* prior to introduction into ES cells we have taken the modified BAC containing the HS -40 major regulatory element deletion as previously described above with heterotypic *lox* sites introduced into the *Dist* and *theta* sequences (see **Figure 11a**) and reintroduced the HS -40 regulatory element into new locations downstream of the α-globin structural genes (see **Figure 11b****).** This involved the use of a further selection marker encoding zeocyin resistance (zeo^{R}) flanked by a pair of heterotypic *frt* sites (*frt-*F5) [N.B. these are different from the *frt-*F3 heterotypic sites flanking the puromycin resistance marker (*puro*^{R}) used in the creation of the HS -40 deletion]. Vectors containing this marker and a single copy of the HS -40 sequence were constructed to insert the HS -40 into the BAC by *red* recombination in *E. coli* into the new locations in both orientations. The BACs were then used for RMGR as previously described.

ES cells were recovered with replacement events (by selection for HPRT⁺ cells) and then further modified by transfection with a FLPe recombinase expression plasmid to remove all the selection markers (*hprt, puro*^{R} and *zeo*^{R}) by recombination between the pairs of *frt, frt-*F3 and *frt*-F5 sites. Selection for HPRT⁻ cells was found to give a high efficiency of co-deletion of the *puro*^{R} and *zeo*^{R} markers, and with no unpredicted rearrangements. Thus ES cell lines were established by the RMGR strategy each with a human α-globin syntenic replacement in which HS -40 was deleted from its normal position and reinserted into the new location with minimal extraneous foreign sequences present (theses only being an *frt* site at the *Dist* gene end of the replacement, an *frt*-F3 site at the original position of the HS -40, an *frt*-F5 site at the new position of the HS -40 and a *lox511* site at the theta gene end of the replacement). These ES cells were then differentiated *in-vitro* to make embryoid bodies and consequently cells of the erythroid lineage derived. This allows a read-out of α-globin expression. Thus far data from one of these relocations has been obtained (see **Table 3** for expression levels of three clones. The interesting result is that reinsertion of the HS -40 into the new position rescues the loss of expression of the original HS -40 deletion and in fact gives a higher level of α-globin expression than found in the non-mutant (i.e. wild type human sequence replacement). This therefore demonstrates the functional integrity of the replacement after complex modification of the sequence. These ES cells are currently being used to make mice to further study the consequences of the regulatory element relocation.

### Further BAC Modification

Another method for BAC modification has also been tested in the context of the RMGR strategy called the 'hit and fix' method for engineering mutations by *red* recombination. This is based on the method of Yang and Sharan 2003. (Yang, Y. and Sharan, S.K. 2003 'A simple two step, "hit and fix" method to generate subtle mutations in BACs using short denatured PCR fragments' Nucleic Acids Research 31, e80). This method allows subtle mutations to be created without any use of selection markers. We have used this to create a 5 nucleotide deletion of the GATA transcription factor binding site within the HS -40 element in the human BAC sequence and this can be currently introduced this into ES cells by RMGR. The 'hit and fix' method combined with RMGR has the potential to allow the introduction of single nucleotide mutations at any position in a BAC sequence, for example to create single nucleotide polymorphisms (SNPs) identified in human populations.

### Discussion

Here we have shown that it is possible to precisely replace a large segment of the mouse genome containing multiple loci with the corresponding, syntenic segment of the human genome. Combining a variety of developments in chromosome engineering, RMGR has significantly extended the principles underpinning RMCE to enable extensive tracts (100s-1000s kb) of mouse genomic DNA to be replaced by the orthologous sequences of other species cloned in BAC libraries. RMGR will therefore be widely applicable and offers several advantages over other approaches to large-scale genome modification. In particular correct genomic replacement using RMGR is genetically selectable, precise and reproducible. This reduces the effort of screening and greatly simplifies the characterization of the chromosomal breakpoints. Minimal extraneous DNA remains to affect the functional integrity of the engineered chromosomal region, provided the locations of the recombination sites are chosen carefully. Once correctly targeted, germ line transmitting ES cells are established, RMGR allows iterative replacement with mutated BACs for detailed functional dissection of genomic sequences. The RMGR strategy will be adaptable for application with other (e.g. PhiC31) (Belteki et al., 2003) site-specific recombination systems to improve the overall efficiency and allow the Cre/lox system to be used to introduce conditional deletions within the human sequence. In its present form the extent of sequence replacement is limited only by the size of the BAC inserts. Recent approaches to construct mega-BACs (Shen et al., 2005) in *E. coli* will therefore extend the size range of replacements possible.

Humanizing the mouse genome by RMGR provides a readily accessible experimental system to study the normal regulation of human genes in an appropriate chromosomal environment, avoiding the notable technical problems and difficulties of interpretation associated with conventional transgenic experiments. Furthermore, it allows the analysis of the human gene rather than its mouse orthologue whose regulation and downstream effects may differ significantly from that of its human counterpart. In the past, it has been claimed that large transgenes (BACs, PACs and YACs) containing all known regulatory elements faithfully recapitulate the timing, cell specificity and levels of human gene expression in mouse models. However, such assertions are rarely, if ever, based on significant numbers of fully mapped, randomly integrated, single copy transgenes and often the conclusion is inappropriately predicated on the ability of the transgene to rescue a lethal phenotype.

By analysing the well characterised α globin regulatory domain by RMGR we could be confident that the structurally intact segment contains all known *cis-*acting elements, at a single copy in a chromosomal position that is appropriate for globin gene expression. Furthermore, by carefully examining gene expression in erythropoiesis and evaluating the resulting, sensitive red cell phenotype, we could accurately assess the degree to which regulation of the human globin genes mimic their normal pattern and levels of expression in the mouse model. We found that under these conditions the human genes are expressed in an appropriate developmental stage- and tissue-specific manner, although the level of expression of the α genes is only about 40% of that of the endogenous mouse α globin genes. This leads to abnormal red blood cells and homozygotes for the human synteny replacement may die prematurely even though they are not frankly anemic. These differences between expression of the human gene in man and mouse could result from changes in the chromosomal environment although the relationship between transcription and nuclear sublocalization remains unclear (Brown et al., 2006). Perhaps more likely, changes in the structure or recognition sequences of the key transcription factors have altered during the evolution of the two species such that the binding or stability of mouse transcription factors on human sequences are suboptimal.

Knowing the phenotype resulting from expression of the normal human α globin cluster in mouse, it is now possible to compare and evaluate the effects of specific mutations introduced into the human sequence. Using RMGR, the only variable is the mutation itself. Such mutations could either be introduced by further rounds of homologous recombination in the humanized ES cells or, as demonstrated here, by recombineering the appropriate mutation into the modified BAC followed by a new round of RMGR at the target region. Again using the α globin cluster as the model we could show that removal of just 1.1kb containing the major regulatory element (HS-40) virtually abolishes human α globin expression consistent with previous extensive observations (Higgs et al., 1998). In homozygotes this deletion mimics the lethal clinical phenotype of *hydrops fetalis* seen in human patients with this degree of α globin deficiency. We have previously shown that removal of the mouse sequence (HS-26), which is the equivalent of the human major regulatory element (HS-40), from the endogenous locus only reduces mouse α globin expression to ~50% of normal and homozygotes for this allele survive with only mild anaemia rather than developing hydrops fetalis (Anguita et al., 2002). This clearly shows that the detailed regulation of human and mouse genes may be quite different and emphasises the point that extrapolating conclusions from studying regulation of an orthologous gene may be misleading when one wishes to determine how a particular human gene is regulated.

It seems likely that the observations set out here represent general recurrent issues that arise when making and analysing mouse models of human gene regulation and genetic disease. RMGR will exclude most of the variables associated with current transgenic experiments but it is still likely that human genes will not be completely faithfully regulated in mouse systems. However, comparing wild type and mutant chromosomal domains iteratively inserted into an appropriate chromosomal environment will allow one to determine more precisely the effect of specific mutations. This will not only be of value when studying previously well characterized loci, but will also enable those studying complex traits to assess the functional role of mutations and SNPs localised within less well characterised large segments of the human genome.

A major problem in contemporary human genetics is to define the functional significance of SNPs identified in the human population. A SNP can have profound regulatory significance. For example Higgs and colleagues have recently shown that a single SNP in the α-globin regulatory domain can result in α-thalasemmia by creating a new promoter site upstream of the α-globin structural genes and reducing α-globin expression (De Gobbi et al. 2006 A regulatory SNP causes a Human Genetic Disease by Creating a New Transcriptional Prpmoter' Science 312, 1215-1217). Combining the ability of to create SNPs in BACs by for example using the 'hit and fix' mutation with RMGR in ES cells to create humanised mice has the potential to establish a robust experimental platform to determine if SNPs have a phenotypic significance. Given the recently improved power of DNA sequencing (through massively parallel sequencing technologies) the numbers of SNPs identified will greatly increase and an experimental means will be required to understand if these are the basis of quantitative trait loci contributing to human disease or drug efficacy. The techniques described herein provide simple and elegant means of generating SNPs, in order that their function can be studied.

As will be appreciated, the methods described herein provide a number of distinct advantages over other prior art methods:
1) genome replacement is genetically selected in ES cells and is thus precise and reproducible;
2) no genetic selection markers are left in place in the genome;
3) equivalent host animal sequences are eliminated from the genome, therefore obviating any requirement to breed with knock-out lines;
   and
4) an unlimited number of replacement events can be made with variously modified vector insert sequences.

### References

Alami, R., Greally, J. M., Tanimoto, K., Hwang, S., Feng, Y. Q., Engel, J. D., Fiering, S., and Bouhassira, E. E. (2000). Beta-globin YAC transgenes exhibit uniform expression levels but position effect variegation in mice. Hum Mol Genet 9,631-636.
Anguita, E., Hughes, J., Heyworth, C., Blobel, G. A., Wood, W. G., and Higgs, D. R. (2004). Globin gene activation during haemopoiesis is driven by protein complexes nucleated by GATA-1 and GATA-2. Embo J 23, 2841-2852.
Anguita, E., Johnson, C. A., Wood, W. G., Turner, B. M., and Higgs, D. R. (2001). Identification of a conserved erythroid specific domain of histone acetylation across the α globin gene cluster. Proceedings of the National Academy of Sciences, USA 98, 12114-12119.
Anguita, E., Sharpe, J. A., Sloane-Stanley, J. A., Tufarelli, C., Higgs, D. R., and Wood, W. G. (2002). Deletion of the mouse α globin regulatory element (HS -26) has an unexpectedly mild phenotype. Blood 100, 3450-3456.
Baer, A., and Bode, J. (2001). Coping with kinetic and thermodynamic barriers: RMCE, an efficient strategy for the targeted integration of transgenes. Curr Opin Biotechnol 12, 473-480.
Belteki, G., Gertsenstein, M., Ow, D. W., and Nagy, A. (2003). Site-specific cassette exchange and germline transmission with mouse ES cells expressing phiC31 integrase. Nat Biotechnol 21, 321-324.
Bethke, B., and Sauer, B. (1997). Segmental genomic replacement by Cre-mediated recombination: genotoxic stress activation of the p53 promoter in single-copy transformants. Nucleic Acids Res 25, 2828-2834.
Brown, J. M., Leach, J., Reittie, J. E., Atzberger, A., Lee-Prudhoe, J., Wood, W. G., Higgs, D. R., Iborra, F. J., and Buckle, V. J. (2006). Coregulated human globin genes are frequently in spatial proximity when active. J Cell Biol 172, 177-187.
Colledge, W. H., Abella, B. S., Southern, K. W., Ratcliff, R., Jiang, C., Cheng, S. H., MacVinish, L. J., Anderson, J. R., Cuthbert, A. W., and Evans, M. J. (1995). Generation and characterization of a delta F508 cystic fibrosis mouse model. Nat Genet 10, 445-452.
Copeland, N. G., Jenkins, N. A., and Court, D. L. (2001). Recombineering: a powerful new tool for mouse functional genomics. Nat Rev Genet 2, 769-779.
De Gobbi, M., Viprakasit, V., Hughes, J. R., Fisher, C., Buckle, V. J., Ayyub, H., Gibbons, R. J., Vemimmen, D., Yoshinaga, Y., de Jong, P., et al. (2006). A regulatory SNP causes a human genetic disease by creating a new transcriptional promoter. Science 312, 1215-1217.
Engle, S. J., Womer, D. E., Davies, P. M., Boivin, G., Sahota, A., Simmonds, H. A., Stambrook, P. J., and Tischfield, J. A. (1996). HPRT-APRT-deficient mice are not a model for lesch-nyhan syndrome. Hum Mol Genet 5, 1607-1610.
Flint, J., Thomas, K., Micklem, G., Raynham, H., Clark, K., Doggett, N. A., King, A., and Higgs, D. R. (1997). The relationship between chromosome structure and function at a human telomeric region. Nature Genetics 15, 252-257.
Flint, J., Tufarelli, C., Peden, J., Clark, K., Daniels, R. J., Hardison, R., Miller, W., Philipsen, S., Tan-Un, K. C., McMorrow, T., et al. (2001). Comparative genome analysis delimits a chromosomal domain and identifies key regulatory elements in the α globin cluster. Human Molecular Genetics 10, 371-382.
Garrick, D., Sharpe, J. A., Arkell, R., Dobbie, L., Smith, A. J., Wood, W. G., Higgs, D.R., and Gibbons, R. J. (2006). Loss of Atrx affects trophoblast development and the pattern of X-inactivation in extraembryonic tissues. PLoS Genet 2, e58. Higgs, D. R., Sharpe, J. A., and Wood, W. G. (1998). Understanding α globin gene expression: a step towards effective gene therapy. Seminars in Hematology 35, 93-104.
Giraldo, P. and Montoliu, L. (2001) size matters: use of YACs, BACs and PACs in transgenic animals. Transgenic Research 10, 83-103.
Hughes, J. R., Cheng, J. F., Ventress, N., Prabhakar, S., Clark, K., Anguita, E., De Gobbi, M., de Jong, P., Rubin, E., and Higgs, D. R. (2005). Annotation of cisregulatory elements by identification, subclassification, and functional assessment of multispecies conserved sequences. Proc Natl Acad Sci U S A 102, 9830-9835.
Kaufman, R. M., Pham, C. T., and Ley, T. J. (1999). Transgenic analysis of a 100-kb human beta-globin cluster-containing DNA fragment propagated as a bacterial artificial chromosome. Blood 94, 3178-3184.
Kielman, M. F., Smits, R., Hof, I., and Bernini, L. F. (1996). Characterization and comparison of the human and mouse Dist1/α-globin complex reveals a tightly packed multiple gene cluster containing differentially expressed transcription units. Genomics 32, 341-351.
Kleinjan, D. A., and van Heyningen, V. (2005). Long-range control of gene expression: emerging mechanisms and disruption in disease. Am J Hum Genet 76, 8-32.
Lee, E. C., Yu, D., Martinez de Velasco, J., Tessarollo, L., Swing, D. A., Court, D. L., Jenkins, N. A., and Copeland, N. G. (2001). A highly efficient Escherichia colibased chromosome engineering system adapted for recombinogenic targeting and subcloning of BAC DNA. Genomics 73, 56-65.
Lee, G. and Saito, I. (1998) Role of nucleotide sequences of loxP spacer region in Cre-mediated recombination. Gene 216, 55-65.
Mansour, S. L., Thomas, K. R., and Capecchi, M. R. (1988). Disruption of the protooncogene int-2 in mouse embryo-derived stem cells: a general strategy for targeting mutations to non-selectable genes. Nature 336, 348-352.
Muyrers, J. P., Zhang, Y., and Stewart, A. F. (2001). Techniques: Recombinogenic engineering--new options for cloning and manipulating DNA. Trends Biochem Sci 26, 325-331.
Nagy A, Rossant J, Nagy R, Abramow-Newerly W, Roder JC. (1993) Derivation of completely cell culture-derived mice from early passage embryonic stem cells. Proc Natl Acad Sci USA. 90, 8424-8428.
Nagy, A. (2003). Manipulating the mouse embryo: a laboratory manual, 3rd edn (Cold Spring Harbor, N.Y.: Cold Spring Harbor Laboratory Press).
Nichols, J., Evans, E. P., and Smith, A. G. (1990). Establishment of germ-linecompetent embryonic stem (ES) cells using differentiation inhibiting activity. Development 110, 1341-1348.
Peterson, K. R., Navas, P. A., Li, Q., and Stamatoyannopoulos, G. (1998). LCRdependent gene expression in beta-globin YAC transgenics: detailed structural studies validate functional analysis even in the presence of fragmented YACs. Hum Mol Genet 7, 2079-2088.
Pászty, C., Mohandas, N., Stevens, M. E., Loring, J. F., Liebhaber, S. A., Brion, C. M., and Rubin, E. M. (1995). Lethal alpha-thalassaemia created by gene targeting in mice and its genetic rescue. Nature Genetics 11, 33-39.
Schaft, J., Ashery-Padan, R., van der Hoeven, F., Gruss, P., and Stewart, A. F. (2001). Efficient FLP recombination in mouse ES cells and oocytes. Genesis 31, 6-10.
Schlake, T., and Bode, J. (1994). Use of mutated FLP recognition target (FRT) sites for the exchange of expression cassettes at defined chromosomal loci. Biochemistry 33, 12746-12751.
Sgaramella, V. and Eridani, S. (2004). Mammalian artificial chromosomes: methods and protocols. Humana Press, New Jersey ISBN 1-55829-096-4; 269pp.
Shen, W., Huang, Y., Tang, Y., Liu, D. P., and Liang, C. C. (2005). A general method to modify BACs to generate large recombinant DNA fragments. Mol Biotechnol 31, 181-186.
Smith, A. J., De Sousa, M. A., Kwabi-Addo, B., Heppell-Parton, A., Impey, H., and Rabbitts, P. (1995). A site-directed chromosomal translocation induced in embryonic stem cells by Cre-loxP recombination. Nat Genet 9, 376-385.
Smith, A. J., Xian, J., Richardson, M., Johnstone, K. A., and Rabbitts, P. H. (2002). Cre-loxP chromosome engineering of a targeted deletion in the mouse corresponding to the 3p21.3 region of homozygous loss in human tumours. Oncogene 21, 4521-4529.
Testa, G., Zhang, Y., Vintersten, K., Benes, V., Pijnappel, W. W., Chambers, I., Smith, A. J., Smith, A. G., and Stewart, A. F. (2003). Engineering the mouse genome with bacterial artificial chromosomes to create multipurpose alleles. Nat-Biotechnol 21, 443-447.
Tronche, F., Casanova, e. Turiault, M. sahly, I. and Kellendonk, C. (2002) When reverse genetics meets physiology: the use of site-specific recombinases in mice. FEBS Letters 529, 116-121.
Tufarelli, C., Stanley, J.A., Garrick, D., Sharpe, J.A., Ayyub, H., Wood, W.G., and Higgs, D.R. (2003). Transcription of antisense RNA leading to gene silencing and methylation as a novel cause of human genetic disease. Nat Genet 34, 157-165.
Valenzuela, D. M., Murphy, A. J., Frendewey, D., Gale, N. W., Economides, A. N., Auerbach, W., Poueymirou, W. T., Adams, N. C., Rojas, J., Yasenchak, J., et al (2003). High-throughput engineering of the mouse genome coupled with highresolution expression analysis. Nat Biotechnol 21, 652-659.
Wiles, M.V., and Keller, G. (1991). Multiple hematopoietic lineages develop from embryonic stem (ES) cells in culture. Development 111, 259-267.
Yagi, T., Ikawa, Y., Yoshida, K., Shigetani, Y., Takeda, N., Mabuchi, I., Yamamoto, T., and Aizawa, S. (1990). Homologous recombination at c-fyn locus of mouse embryonic stem cells with use of diphtheria toxin A-fragment gene in negative selection. Proc Natl Acad Sci U S A 87, 9918-9922.
Yang, Y., and Seed, B. (2003). Site-specific gene targeting in mouse embryonic stem cells with intact bacterial artificial chromosomes. Nat Biotechnol 21, 447-451.
Yu, Y., and Bradley, A. (2001). Engineering chromosomal rearrangements in mice. Nat Rev Genet 2, 780-790.
Zheng, B., Sage, M., Sheppeard, E.A., Jurecic, V., and Bradley, A. (200). Engineering mouse chromosomes with Cre-loxP: range, efficiency, and somatic applications. Mol Cell Biol 20, 648-655.

**Table 1: A: Hematologic data and ratios of human to mouse α globin mRNA levels in adult blood from mice heterozygous for either the intact human BAC (lines 1 and 3, with the Hprt gene present (+H) or after its removal (-H) with Flpe recombinase) or one from which the major regulatory element has been deleted (Δ -40), before (+H, +P) and after removal (-H, -P) of the Hprt and puromycin resistance genes.). Equivalent results from normal mice and those carrying a deletion of the structural α globin genes are provided for comparison. n= 3-11 animals. B: Hematologic data on adult blood from mice homozygous for the two human BAC lines, with the Hprt mini-gene present or after it was removed. n=2-5 animals.**

| αH/αM mRN | | | | | | |
|---|---|---|---|---|---|---|
| | Hb g/dl | MCV Fl | MCH pg | Retics % | Real-time PCR | RPA |
| A | | | | | | |
| Human BAC Line 1 (+H) | 15.2±1.0 | 43.2±2.4 | 15.6±0.4 | 1.7 ± 1.1 | 0.43±0.07 | 0.39±0 |
| Human BAC line 1 (-H) | 15.0±0.8 | 45.8±1.1 | 15.7±0.6 | 1.9±0.5 | | 0.37±0 |
| Human BAC line 3 (+H) | 15.5±3.2 | 44.7±3.1 | 15.8±1.2 | 0.8±0.7 | 0.39±0.04 | 0.49±0. |
| Human BAC line 3 (+H) | 14.4±0.7 | 44.8±1.5 | 15.3±0.5 | 1.6±0.6 | | 0.49±0. |
| Δ -40 BAC (+H, +P) | 13.6±0.4 | 36.6±1.3 | 14.4±1.3 | 2.3±0.5 | 0.016±0.005 | 0.016± |
| Δ -40 BAC (-H, -P) | 14.2±1.5 | 39.5±3.5 | 12.9±0.8 | 2.8±0.7 | | |
| Normal mouse | 15.9±1.1 | 45.7±2.4 | 16.5±0.3 | 1.4±1.0 | | |
| -/αα | 11.8±0.9 | 36.7±1.5 | 13.7±0.7 | 3.9±1.0 | | |
| | | | | | | |

| B | | | | | | |
|---|---|---|---|---|---|---|
| Human BAC line 1 (+H) | 15.2±0.6 | 39.8±0.8 | 13.1±0.3 | 5.8±2.7 | | |
| Human BAC line 3 (+H) | 14.4±0.5 | 40.0±0.1 | 13.3±0.2 | 6.7±1.8 | | |
| Human BAC line 3 (-H) | 14.6±2.0 | 40.0±1.4 | 13.1±0.3 | 7.0±4.5 | | |

**Table 2: Distinguishing in-cis and in-trans Dist/θ double targeted ES cell clones. Data obtained from six independently derived Dist/θ double targeted clones. Two frequency classes are obtained after co-transfection with a plasmid vector pBS-loxP/Hprt-^{Δ5'}/lox511 and the Cre recombinase expressing plasmid.**

| Cell lines | Frequency of HAT^{R} colonies |
|---|---|
| 2/B-6 | 3 x 10⁻⁶ |
| 2/H-1 | 2.5 x 10⁻⁶ |
| 2/H-10 | 2.6 x 10⁻⁶ |
| 2/C-7 | 2 x 10⁻⁷ |
| 2/C-10 | 2.4 x 10⁻⁷ |
| 2/G-4 | 2.5 x 10⁻⁷ |

**TABLE 3 α-globin expression levels**

| **SAMPLE** | **Ha** | **Ma** | **Mb** | **Ha/Mb** | **Ma/Mb** | **Human relative to mouse** |
|---|---|---|---|---|---|---|
| **HWT** | 15.99 | 53.2 | 15.82 | 1.64 | 3.36 | 0.49 |
| | 24.3 | 59.09 | 15.28 | 1.59 | 3.87 | 0.41 |
| | 24.71 | 50.32 | 15.57 | 1.59 | 3.23 | 0.49 |
| **AVERAGE** | **24.96** | **54.20** | **15.56** | **1.60** | **3.49** | **0.46** |
| **40 del** | 0.00E+00 | 16.18 | 4.65 | 0.00 | 3.48 | 0.00 |
| | 0.00E+00 | 17.58 | 4.67 | 0.00 | 3.76 | 0.00 |
| | 0.00E+00 | 16.56 | 4.71 | 0.00 | 3.52 | 0.00 |
| **AVERAGE** | **0.00** | **16.77** | **4.68** | **0.00** | **3.59** | **0.00** |
| **rrt2a** | 7.72 | 13.61 | 4.8 | 1.61 | 2.84 | 0.57 |
| **c8** | 7.98 | 13.76 | 4.88 | 1.64 | 2.82 | 0.58 |
| | 8.01 | 14.57 | 4.85 | 1.65 | 3.00 | 0.55 |
| **AVERAGE** | **7.90** | **13.98** | **4.84** | **1.63** | **2.89** | **0.57** |
| **rrt2a** | 1.80E-01 | 4.38E-01 | 3.01E-01 | 0.60 | 1.46 | 0.41 |
| **a8** | 2.43E-01 | 3.36E-01 | 3.07E-01 | 0.79 | 1.09 | 0.72 |
| | 2.06E-01 | 3.19E-01 | 2.96E-01 | 0.70 | 1.08 | 0.65 |
| **AVERAGE** | **0.21** | **0.36** | **0.30** | **0.70** | **1.21** | **0.59** |
| **rrt2a** | 16.26 | 20.66 | 7.04 | 2.31 | 2.93 | 0.79 |
| **c10** | 18.43 | 18.35 | 6.86 | 2.69 | 2.67 | 1.00 |
| | 16.74 | 19.23 | 6.88 | 2.43 | 2.80 | 0.87 |
| **AVERAGE** | **17.14** | **19.41** | **6.93** | **2.48** | **2.80** | **0.89** |

| | | | | | | |
|---|---|---|---|---|---|---|
| α-globin exprossion levels (Ha a human α globin: Ha a mouse α-globin) in *in*-*vitro* derivated ES cells with normal human sequence (HWT) replacement human sequence replacement with H8 -40 deletion (40 del), human sequence remplacement with HS -40 relocated derivation of α-globin structural gene (rrt2a-3 clones c8, a8, 010) | | | | | | |

## Claims

1. An *in vitro* method of generating a non-human animal cell in which a single large fragment of at least 50 kb of a region of a chromosome from the cell's genome has been replaced by a corresponding region from a chromosome of a different organism, comprising the steps of:
a) providing an isolated non-human animal cell in the genome of which two site-specific recombination sites have been introduced that are not capable of recombining with each other, and which flank a portion of genome to be replaced with a corresponding region from a chromosome of a different organism,
b) providing a vector comprising said equivalent portion of genomic nucleic acid from the different organism flanked by the same two site-specific recombination sites that flank the portion of genome to be replaced in the cell; wherein the vector is a bacterial artificial chromosome (BAC), a PI artificial chromosome (PAC), a yeast artificial chromosome (YAC) or a mammalian artificial chromosome (MAC);
c) bringing said genome and said vector into contact *in vitro*, in combination with a site specific recombinase which is capable of recognising said two recombinase sites, such that under appropriate conditions a recombination event can occur which results in the equivalent portion of nucleic acid in the vector, replacing the corresponding portion of genome from the cell; and
d) isolating a cell which comprises the replaced portion of nucleic acid.

2. An *in vitro* method according to claim 1 wherein in step
(a) the non-human animal cell further comprises a first inactive portion of a marker gene flanked 3' or 5' by a further second site-specific recombinase site; in step
(c), upon replacement of the portion of genome, said first and second inactive portions of the marker gene are brought into functional association, such that the replacement reaction can be selected for by use of a selection medium, whereby only cells which possess a functionally active marker gene and have been subject to the replacement reaction are able to survive in said selection medium;
and further comprising the step:
(e) contacting said cell of step (d) with a recombinase that is capable of recognising said further second site-specific recombinase sites flanking said functional marker gene, such that site-specific recombination can occur, so as to delete the marker gene from the genome.

3. The method according to claim 2 wherein the first inactive portion of the maker gene is a 5' or 3' defective portion of a functional hypoxanthine-phosphoribosyltransferase (HPRT) mini-gene, and the second inactive portion is a respective 3' or 5' defective further portion of said HPRT mini-gene.

4. The method according to any preceding claim wherein the replaced portion of genome comprises a deletion, mutation, or SNP, as compared to wild type sequence.

5. The method according to any of claims 1-4 wherein the nucleic acid which is used for replacement is human nucleic acid.

## Patentansprüche

1. In-vitro-Verfahren zum Erzeugen einer nichtmenschlichen tierischen Zelle, in der ein einzelnes großes Fragment mit einer Größe von mindestens 50 kb einer Region eines Chromosoms von dem Genom der Zelle durch eine entsprechende Region von einem Chromosom eines unterschiedlichen Organismus ersetzt worden ist, umfassend die folgenden Schritte:
a) Bereitstellen einer isolierten nichtmenschlichen tierischen Zelle, in deren Genom zwei ortsspezifische Rekombinationsstellen eingeführt worden sind, die nicht miteinander rekombinieren können und die einen Genomabschnitt flankieren, der durch eine entsprechende Region von einem Chromosom eines unterschiedlichen Organismus ersetzt werden soll,
b) Bereitstellen eines Vektors, der den entsprechenden genomischen Nukleinsäureabschnitt von einem unterschiedlichen Organismus umfasst, der von denselben beiden ortsspezifischen Rekombinationsstellen flankiert ist, die den in der Zelle zu ersetzenden Genomabschnitt flankieren; wobei es sich bei dem Vektor um ein künstliches Bakterienchromosom (BAC), ein künstliches P1-Chromosom (PAC), ein künstliches Hefechromosom (YAC) oder ein künstliches Säugetierchromosom (MAC) handelt;
c) in-vitro-Inkontaktbringen des Genoms und des Vektors in Kombination mit einer ortsspezifischen Rekombinase, die die beiden Rekombinasestellen erkennen zu vermag, so dass unter entsprechenden Bedingungen ein Rekombinationsereignis stattfinden kann, das dazu führt, dass der äquivalente Nukleinsäureabschnitt in dem Vektor den entsprechenden Genomabschnitt der Zelle ersetzt; und
d) Isolieren einer Zelle, die den ersetzten Nukleinsäureabschnitt umfasst.

2. In-vitro-Verfahren nach Anspruch 1, wobei in Schritt (a) die nichtmenschliche tierische Zelle weiterhin einen ersten inaktiven Abschnitt eines Markergens, der 3'- oder 5'-seitig von einer weiteren zweiten ortsspezifischen Rekombinasestelle flankiert ist, umfasst;
in Schritt (c) bei Ersetzen des Genomabschnitts die ersten und zweiten inaktiven Abschnitte des Markergens funktionell miteinander assoziiert werden, so dass durch Verwendung eines Selektionsmediums die Ersatzreaktion selektiert werden kann, wobei nur Zellen, die ein funktionell aktives Markergen aufweisen und die der Ersatzreaktion unterzogen worden sind, fähig sind, in dem Selektionsmedium zu überleben;
und das weiterhin den folgenden Schritt umfasst:
(e) Inkontaktbringen der Zelle von Schritt (d) mit der Rekombinase, die fähig ist, die weiteren zweiten ortsspezifischen Rekombinasestellen, die das funktionelle Markergen flankieren, zu erkennen, so dass eine ortsspezifische Rekombination stattfinden kann, um das Markergen aus dem Genom zu deletieren.

3. Verfahren nach Anspruch 2, wobei es sich bei dem ersten inaktiven Abschnitt des Markergens um einen 5'- oder 3'-seitig defizienten Abschnitt eines funktionellen Hypoxanthinphosphoribosyltransferase(HPRT)-Minigens und bei dem zweiten inaktiven Abschnitt um einen entsprechenden 3'- oder 5'-seitig defizienten weiteren Abschnitt des HPRT-Minigens handelt.

4. Verfahren nach einem vorhergehenden Anspruch, wobei der ersetzte Genomabschnitt eine Deletion, eine Mutation oder einen SNP im Vergleich zu der Wildtypsequenz umfasst.

5. Verfahren nach einem der Ansprüche 1-4, wobei es sich bei der für den Ersatz verwendeten Nukleinsäure um humane Nukleinsäure handelt.

## Revendications

1. Procédé *in vitro* de production d'une cellule animale non humaine, dans laquelle un gros fragment unique d'au moins 50 kb d'une région d'un chromosome provenant du génome de la cellule a été remplacé par une région correspondante provenant d'un chromosome d'un organisme différent, comprenant les étapes de :
a) fourniture d'une cellule animale non humaine isolée, dans le génome de laquelle deux sites de recombinaison spécifiques de site ont été introduits, qui ne sont pas capables de se recombiner l'un avec l'autre, et qui flanquent une portion du gène à remplacer avec une région correspondante provenant d'un chromosome d'un organisme différent,
b) fourniture d'un vecteur comprenant ladite portion équivalente d'un acide nucléique génomique provenant de l'organisme différent flanqué des mêmes deux sites de recombinaison spécifique de site que ceux qui flanquent la portion du génome à remplacer dans la cellule ; le vecteur étant un chromosome artificiel bactérien (BAC), un chromosome artificiel de P1 (PAC), un chromosome artificiel de levure (YAC) ou un chromosome artificiel de mammifère (MAC) ;
c) mise dudit génome et dudit vecteur en contact *in vitro*, en combinaison avec une recombinase spécifique de site, qui est capable de reconnaître lesdits deux sites de recombinase, de telle sorte que, dans des conditions appropriées, un événement de recombinaison puisse se produire, qui conduit à la portion équivalente de l'acide nucléique dans le vecteur, en remplaçant la portion correspondante du génome provenant de la cellule ; et
d) isolement d'une cellule qui comprend la portion remplacée de l'acide nucléique.

2. Procédé *in vitro* selon la revendication 1, dans lequel, dans l'étape (a), la cellule animale non humaine comprend en outre une première portion inactive d'un gène marqueur flanqué en 3' ou en 5' par un deuxième site supplémentaire de recombinaison spécifique de site ; dans l'étape (c), après remplacement de la portion du génome, lesdites première et deuxième portions inactives du gène marqueur sont mises en association fonctionnelle, de telle sorte que la réaction de remplacement puisse être choisie par utilisation d'un milieu de sélection, ce en conséquence de quoi seules sont capables de survivre dans ledit milieu de sélection les cellules qui possèdent un gène marqueur fonctionnellement actif et qui ont été soumises à la réaction de remplacement ; et comprenant en outre l'étape (e) de mise en contact de ladite cellule de l'étape (d) avec une recombinase qui est capable de reconnaître lesdits deuxièmes sites supplémentaires de recombinaison spécifiques de site flanquant ledit gène marqueur fonctionnel de telle sorte qu'une recombinaison spécifique de site puisse se produire, de façon à déléter du génome le gène marqueur.

3. Procédé selon la revendication 2, dans lequel la première portion inactive du gène marqueur est une portion défective en 5' ou en 3' d'un minigène fonctionnel de l'hypoxanthine-phosphoribosyltransférase (HPRT), et la deuxième portion inactive est une portion défective en 3' ou 5' supplémentaire respective dudit minigène HPRT.

4. Procédé selon l'une quelconque des revendications précédentes, dans lequel la portion remplacée du génome comprend une délétion, une mutation ou un SNP par comparaison avec la séquence de type sauvage.

5. Procédé selon l'une quelconque des revendications 1 à 4, dans lequel l'acide nucléique qui est utilisé pour le remplacement est un acide nucléique humain.
